# FASCICULE DE BREVET EUROPEEN

(11) **EP 3 692 367 B1**
(45) Date de publication et mention de la délivrance du brevet: **02.07.2025**
(21) Numéro de dépôt: 18782435.4
(22) Date de dépôt: 04.10.2018
(51) Int. Cl.: G01N 33/543, G01N 33/558, G01N 33/94, G01N 21/64, G01N 33/58, G01N 21/25, G01N 21/84, G01N 15/06

(54) **MOYEN DE DIAGNOSTIC POUR LA DÉTECTION ET/OU LA QUANTIFICATION D'UNE PLURALITÉ D'ANALYTES PRÉSENTS DANS UN ÉCHANTILLON**
VORRICHTUNG ZUM OPTISCHEN AUSLESEN "A LA CARTE" VON EINEM ABNEHMBAREN FESTEN TRÄGER ZUM NACHWEIS UND/ODER ZUR QUANTIFIZIERUNG VON ANALYTEN IN EINER PROBE
DEVICE FOR OPTICAL READ-OUT "A LA CARTE" OF A REMOVABLE SOLID SUPPORT FOR DETECTING AND/OR QUANTIFYING ANALYTES IN A SAMPLE

(30) Priorité: 04.10.2017 BE 201705705; 04.10.2017 BE 201705706; 04.10.2017 BE 201705707; 04.10.2017 BE 201705708; 04.10.2017 BE 201705709
(43) Date de publication de la demande: 12.08.2020
(73) Titulaire: Unisensor, 4120 Neupré (BE)
(72) Inventeur: CHABOTTAUX, Vincent, 4020 Wandre (BE); GLOUDEN, Thomas, 4000 Liège (BE); GRANIER, Benoit, 4120 Neupré (BE)
(74) Mandataire: Gevers Patents
(86) Numéro de dépôt international: PCT/EP2018/076993
(87) Numéro de publication internationale: WO 2019/068806

(56) Documents cités:
- EP-A1- 1 712 914
- WO-A1-2017/075649
- US-A1- 2014 024 016
- LE CONSEIL DES COMMUNAUTÉS EUROPÉENNES: "Directive 93/42/CEE du Conseil, du 14 juin 1993, relative aux dispositifs médicaux", 12 July 1993 (1993-07-12), EUR-lex, XP055101562, Retrieved from the Internet <URL:http://eur-lex.europa.eu/LexUriServ/LexUriServ.do?uri=CELEX:31993L0042:FR:HTML> [retrieved on 20140212]
- ANONYMOUS: "Carbadox", 12 November 2020 (2020-11-12), pages 1 - 40, XP093106643, Retrieved from the Internet <URL:https://pubchem.ncbi.nlm.nih.gov/compound/Carbadox> [retrieved on 20231128]
- BAKKER E P: "Ionophore Antibiotics: introduction", MECHANISM OF ACTION OF ANTIBACTERIAL AGENT, 1 January 1979 (1979-01-01), pages 67 - 68, XP093106639, Retrieved from the Internet <URL:https://link.springer.com/chapter/10.1007/978-3-642-46403-4_5> [retrieved on 20231128]
- THOMAS P. O'CONNOR: "SNAP Assay Technology", TOPICS IN COMPANION ANIMAL MEDICINE, vol. 30, no. 4, 1 December 2015 (2015-12-01), AMSTERDAM, NL, pages 132 - 138, XP055473890, ISSN: 1938-9736, DOI: 10.1053/j.tcam.2015.12.002
- KNECHT B G ET AL: "AUTOMATED MICROARRAY SYSTEM FOR THE SIMULTANEOUS DETECTION OF ANTIBIOTICS IN MILK", ANALYTICAL CHEMISTRY, AMERICAN CHEMICAL SOCIETY, US, vol. 76, no. 3, 1 February 2004 (2004-02-01), pages 646 - 654, XP001047318, ISSN: 0003-2700, DOI: 10.1021/AC035028I
- NADEZHDA A. TARANOVA ET AL: "Integration of lateral flow and microarray technologies for multiplex immunoassay: application to the determination of drugs of abuse", MIKROCHIMICA ACTA, vol. 180, no. 11-12, 13 July 2013 (2013-07-13), pages 1165 - 1172, XP055152769, ISSN: 0026-3672, DOI: 10.1007/s00604-013-1043-2

## Description

### Domaine technique

La présente invention se rapporte à un moyen de diagnostic immuno-chromatographique pour la détection et/ou la quantification respective, simultanée et spécifique d'une pluralité d'analytes présents dans un échantillon essentiellement liquide comprenant:
- au moins un mélange réactionnel contenant des molécules biologiques de reconnaissance et/ou des ligands compétiteurs marqués avec au moins une molécule de visualisation ; et
- au moins un système récupérateur sous la forme d'un support solide auquel se trouvent fixés des ligands compétiteurs et/ou des molécules biologiques de reconnaissance en des emplacements de récupération distincts et connus, de manière à identifier par la localisation desdits emplacements de récupération sur ledit support, lesdits analytes présents dans ledit échantillon.

### Arrière-plan technologique

De nos jours, l'intérêt pour des moyens de diagnostic permettant la détection et/ou la quantification respective, simultanée et spécifique d'analytes présents dans un échantillon est grandissant, et ce particulièrement dans le domaine des produits alimentaires de même que dans le domaine médical. Par exemple, des moyens de diagnostic de type ELISA comprenant des enzymes sont décrits dans O'Connor et al. 2015 et Knecht et al. 2004 et des moyens de diagnostic avec une détection reposant sur des nanoparticules d'or sont décrits dans les documents US2012/0184462 et WO2017/075649.

En effet, nous devons continuellement faire face à l'émergence de nouveaux problèmes de santé publique contre lesquels des solutions rapides et efficaces de diagnostic doivent être élaborées en vue de fournir un traitement approprié. Par exemple, chaque année à travers le monde, environ 60 000 intoxications humaines sont liées aux toxines produites par des algues (y compris les cyanotoxines d'eau douce), avec une mortalité totale d'approximativement 1,5 %. Les biotoxines marines (également appelées phycotoxines) sont produites par certaines espèces de phytoplancton et sont susceptibles de s'accumuler dans diverses espèces marines, par exemple dans les poissons, crabes ou bivalves filtreurs (coquillages) tels que moules, huitres, coquilles Saint-Jacques et palourdes. Si l'homme consomme des quantités importantes de coquillages contaminés, il peut être victime d'une grave intoxication. Il est donc crucial de posséder des moyens de diagnostic rapides et efficaces pour détecter ces biotoxines marines, par exemple via une analyse du sang ou des urines.

Des moyens de diagnostic tels que définis plus haut peuvent également être utilisés pour la détection et la quantification de virus responsables de pathologies très diverses. De tels moyens de diagnostic permettraient : (1) d'apporter la preuve de l'origine virale des signes cliniques observés et diagnostiquer le virus en cause (par exemple des hépatites ou l'herpès) et de suivre l'évolution biologique de l'infection (par exemple via la quantification du virus dans le sang : VIH, VHB, VHC) ; (2) de suivre une évolution biologique de l'infection (par exemple le VIH ou l'hépatite B) ; (3) de permettre une décision thérapeutique et juger de l'efficacité des traitements antiviraux (par exemple pour le traitement d'une infection à cytomégalovirus par du ganciclovir) ; (4) de prévenir la transmission d'infections virales à l'occasion du don de sang, d'organes et de tissus ; (5) d'apprécier l'état immunitaire (par exemple dans le cas de la rubéole) ; (6) d'étudier les marqueurs sériques en population (par exemple lors d'enquêtes de prévalence ou d'études épidémiologiques). De manière générale, le diagnostic médical vise une étendue maximale des paramètres à détecter pour mieux cibler le traitement et le type de soin à fournir au patient ce qui limite notamment les effets secondaires souvent mal connus.

Par ailleurs, dans le domaine alimentaire et plus particulièrement dans l'industrie du lait, la surveillance et le contrôle des produits imposent d'effectuer des tests le plus en amont possible de leur fabrication. Idéalement ces tests doivent être réalisés sur le lieu de production des matières premières ou sur leur lieu de leur transformation. Ces tests de dépistages, également appelés tests de « screening » sont particulièrement conçus pour détecter la présence et la quantité de certains analytes dont des contaminants chimiques (par exemple des résidus antibiotiques et des toxines), des protéines (par exemple des allergènes) ou des pathogènes (par exemple des virus, des parasites ou des bactéries). La multiplication des normes sanitaires et la volonté d'une meilleure traçabilité des produits alimentaires imposent une multiplication des analytes à tester ainsi que de connaitre le plus précisément possible leurs classes (identifications des familles, classes et du composé distinct) et leurs quantités par rapport aux limites maximales autorisées dans chaque matrices. Par ailleurs, le lait provenant de nombreux endroits très variés à travers le monde, il est difficile de déterminer précisément les contaminants pouvant être retrouvés dans le lait en fonction du lieu de production, tant les pratiques sont différentes d'un endroit de la planète à l'autre. En effet, l'origine des denrées alimentaires ainsi que les pratiques locales de production associées ne sont pas toujours connues, ce qui oblige à détecter en large spectre de composés, le plus large possible couvrant tout ce qui peut se trouver dans l'échantillon à analyser.

En particulier, le secteur agroalimentaire est intéressé par un moyen de diagnostic permettant d'envisager en une seule opération l'analyse de composés appartenant à des classes différentes pouvant avoir des propriétés physico-chimiques fondamentalement différentes, au sein d'une même famille d'analytes ou non, et présents simultanément dans un échantillon donné. Par exemple, le type et le nombre d'antibiotiques qui peut être administré à des animaux peut varier selon qu'il s'agit d'une application thérapeutique ou prophylactique, selon l'espèce animale, le germe à combattre, les pratiques vétérinaires, la législation en vigueur, les moyens disponibles ou encore les régions géographiques. Dans le cas de certains traitements particuliers, un mélange de médicament peut être utilisé. En règle générale, le praticien utilise seul ou en combinaison des produits antibiotiques choisis parmi tous les composés commercialement disponibles selon son appréciation de la meilleure efficacité.

Les principales classes d'antibactériens et d'antibiotiques sont : les pénicillines et céphalosporines, les tétracyclines, les sulfamides, les aminoglycosides et aminocyclitols, les macrolides, les chloramphénicols ou autres peptides, ionophores, nitrofurannes, quinolones, carbadox, etc., chacune de ces classes regroupant un ensemble très vaste de composés chimiquement différents.

La présence de telles molécules dans les produits laitiers peut avoir un impact négatif majeur sur la rentabilité du procédé industriel impliquant une fermentation (fromage, yoghourt,...) à partir du lait frais.

De plus, l'utilisation, parfois intensive, d'antibiotiques en médecine vétérinaire et en production agricole pourrait être à l'origine de l'émergence de souches bactériennes devenues résistantes aux antibiotiques. Pour préserver la santé humaine et légiférer en la matière, de nombreux pays ont établi des limites maximales autorisées (MRL) pour les résidus antibiotiques dans les denrées alimentaires. Ces MRL fixent la limite entre un échantillon positif et un échantillon négatif, c'est-à-dire entre un échantillon refusé et un échantillon accepté.

Il est important que les méthodes de dépistage faisant appel à un moyen de diagnostic (1) puissent couvrir la détection simultanée d'un maximum de composés, les tests de dépistage devant donc préférentiellement et logiquement être des tests multi-analytes, (2) permettent de connaitre les classes auxquelles appartiennent les composés retrouvés dans un échantillon positif de manière à pouvoir orienter directement vers la méthode de confirmation adéquate, et (3) ne puisse pas donner de résultats de type « faux négatif » car ceux-ci échapperont dès lors à l'analyse et ne seront pas confirmés ultérieurement.

### Etat de la technique

Un moyen de diagnostic tel qu'indiqué au début est connu. En effet, le document antérieur EP1712914 divulgue un moyen de diagnostic immuno-chromatographique pour la détection et/ou la quantification respective, simultanée et spécifique d'une pluralité d'analytes présents dans un échantillon essentiellement liquide comprenant:
- au moins un mélange réactionnel contenant des molécules biologiques de reconnaissance et/ou des ligands compétiteurs marqués avec au moins une molécule de visualisation ; et
- au moins un système récupérateur sous la forme d'un support solide auquel se trouvent fixés des ligands compétiteurs et/ou des molécules biologiques de reconnaissance en des emplacements de récupération distincts et connus, de manière à identifier par la localisation desdits emplacements de récupération sur ledit support, lesdits analytes présents dans ledit échantillon.

Plus précisément, ce document antérieur fournit un moyen de diagnostic permettant de détecter simultanément un ensemble de composés pouvant appartenir à au moins deux classes distinctes d'analytes et de caractériser la classe à laquelle appartient effectivement un composé détecté, et ce en démontrant la compatibilité technique et pratique de réunir en un seul et même procédé au moins deux mécanismes de détection sans que le fonctionnement de l'un d'entre eux ne puisse interférer avec le fonctionnement de l'autre. En outre, un moyen de diagnostic selon le document EP1712914 démontre la faisabilité technique d'un dosage multi-analytes qui peut s'opérer rapidement, par exemple en moins de 10 minutes, et en une seule et même étape d'analyse au départ d'un seul et même échantillon.

Pratiquement, le procédé de mise en œuvre du moyen de diagnostic selon le document EP1712914 est caractérisé par les étapes suivantes :
- mise en contact d'un mélange réactionnel prédéterminé avec un échantillon à caractériser pour obtenir une solution qui est incubée à 50°C pendant 3 minutes ;
- trempage du système récupérateur défini plus haut dans la solution obtenue et incubation pendant 3 minutes ;
- interprétation quantitative et qualitative du résultat sur le système récupérateur au moyen d'un dispositif de lecture optique.

Selon ce document antérieur, c'est le positionnement des éléments récupérateurs (des ligands compétiteurs) qui permettra d'identifier le type de contamination. Par exemple, selon exemple du document EP1712914 qui correspond au dosage simultané des tétracyclines, β-lactames et sulfamides, chaque élément récupérateur est disposé sous la forme d'une ligne de capture, chacune d'entre elles étant disposée successivement l'une derrière l'autre en se référant au sens de migration du liquide (correspondant à un mélange réactionnel mis en contact avec un échantillon). Selon une modalité préférée de ce moyen de diagnostic, les zones de captures comprenant les éléments récupérateurs des β-lactames, des tétracyclines et des sulphadiméthoxine sont disposées respectivement à un premier, à un deuxième et à un troisième niveau en se référant au sens de migration du liquide.

L'interprétation des résultats selon un tel moyen de diagnostic doit se faire de manière inverse et est basé sur le principe de compétition qui exploite la reconnaissance des composés recherchés vis-à-vis d'un ligand compétiteur et/ou d'une molécule biologique de reconnaissance. Plusieurs cas peuvent se présenter quand les éléments récupérateurs fixés sur le système de récupération sont les ligands compétiteurs :
- soit le composé recherché est présent dans l'échantillon et va alors se lier aux molécules biologiques de reconnaissance présentes dans un mélange réactionnel qui ne seront par conséquent plus libres de se lier aux molécules compétitrices fixées au système récupérateur. Le résultat sera alors positif et présentera un marquage qui est absent ;
- soit le composé recherché est absent de l'échantillon, les molécules biologiques de reconnaissance présentes dans un mélange réactionnel étant donc libres de se lier aux molécules compétitrices fixées au système récupérateur. Le résultat sera alors négatif et présentera un marquage qui est présent.

Malheureusement, un moyen de diagnostic selon le document EP1712914 ne permet la détection et/ou la quantification que d'un nombre limité d'analytes présents dans un échantillon et ne peut donc pas être considérés comme étant réellement un moyen de diagnostic multi-analytes. Plus précisément, le moyen de diagnostic selon le document EP1712914 permet de détecter des composés appartenant à trois classes distinctes d'antibiotiques uniquement, à savoir les β-lactames, les tétracyclines et les sulfamides.

Par conséquent, même si les β-lactames, les tétracyclines et les sulfamides constituent effectivement des classes d'analytes pouvant être considérées comme étant différentes, ce document antérieur permet de détecter uniquement des antibiotiques. Ainsi, un moyen de diagnostic selon ce document antérieur ne permet certainement pas de détecter et/ou de quantifier des analytes, comme les antibactériens, les toxines, les hormones, les pathogènes, les adultérants ou encore les allergènes.

En effet, les secteurs concernés tels que le secteur agroalimentaire et le secteur médical sont demandeurs d'une analyse la plus complète possible et qui puisse de préférence identifier un maximum de composés. Il est plus pratique et plus économique de réaliser un seul test multiple à partir d'un seul échantillon plutôt que de devoir réaliser un test particulier pour chaque composé ou pour un petit groupe seulement de composés, à savoir 2 ou 3 composés maximum tel que c'est le cas avec un moyen de diagnostic selon le document antérieur EP1712914.

Comme décrit plus haut, ce document antérieur comprend un système récupérateur présentant des zones de capture (éléments récupérateurs fixés) sous la forme de lignes disposées les unes derrières les autres et perpendiculairement au sens de migration du liquide, une incompatibilité technique est rencontrée lorsque l'homme de métier tente de disposer un nombre plus élevé de zones de capture simultanément sur le système récupérateur, et ce à cause de (1) la taille restreinte de la zone de tests, (2) la quantité plus importante de réactifs à déposer sur les lignes successives (favorisant un bruit de fond et des inter-réactivités plus importantes) et (3) un manque de précision lors de l'interprétation des résultats avec une analyse visuelle ou instrumentale qui est longue et complexe. Il devient donc difficile voire impossible de délimiter les différentes zones de capture les unes des autres et donc de distinguer les différents analytes les uns des autres.

La publication de Taranova (Taranova et al., 2013) tente de solutionner les déficiences du document EP1712914 en combinant l'immuno-chromatographie et la technologie du « microarray ». En effet, en vue d'augmenter le nombre d'analytes pouvant être détectés/quantifiés en un seul test, le document de Taranova propose de disposer les emplacements de récupération sur le support solide selon un arrangement matriciel en deux dimensions. De cette façon, le support solide du moyen de diagnostic immuno-chromatographique selon Taranova présente un microarray composé de 32 antigènes (ligands compétiteurs) fixés sous la forme de points (emplacements de récupération). Malheureusement, un moyen de diagnostic selon ce document antérieur permet la détection et la quantification uniquement de quatre analytes, à savoir l'amphétamine, la benzoylecgonine, la méthamphétamine et la morphine, qui sont reconnus comme étant des drogues d'abus. En effet, selon Taranova *et al*., huit emplacements de récupération sous la forme de points sont prévus sur le support solide pour la détection et la quantification d'un seul analyte. Précisément, pour la détection et/ou la quantification d'un analyte donné, huit points comprenant des antigènes (ligands compétiteurs) spécifiques de cet analyte sont fixés sur le support solide, les huit points permettant d'identifier l'analyte donné étant disposés selon l'axe perpendiculaire au sens de migration du liquide. Par conséquent, selon ce document antérieur, il ne s'agit pas de 32 antigènes différents qui permettent de détecter 32 analytes différents qui sont fixés sur le support solide, mais bien uniquement de quatre antigènes différents reproduits huit fois qui sont spécifiques de quatre analytes différents. Ainsi, l'identification des analytes ne se fait que selon une seule dimension, les huit emplacements de récupération disposés selon l'axe perpendiculaire au sens de migration étant identiques, à savoir qu'ils comprennent des antigènes spécifiques d'un seul analyte. Selon ce document antérieur, l'arrangement des emplacements de récupération sous la forme de points se fait selon des rangées de points, chaque rangée correspondant à un analyte donné, et non selon un réel arrangement matriciel en deux dimensions.

Ainsi, les moyens de diagnostic immuno-chromatographiques de l'état de la technique rencontrent à ce stade une limite importante à l'efficacité qui est caractérisée par l'absence d'un test réellement multi-analytes qui est rapide et pratique et qui permet une détection et/ou une quantification d'analytes qui est :
- spécifique, c'est-à-dire qui parvient à distinguer des analytes de classes différentes, et
- universelle, c'est-à-dire qui est applicable pour la plupart des substances utiles à analyser dans les domaines de l'agro-alimentaire et du diagnostic médical telles que les résidus médicamenteux (par exemple les antibiotiques et les antibactériens), les toxines, les hormones, les pathogènes, les adultérants ou encore les allergènes .

### But de l'invention

L'invention a pour but de pallier les inconvénients de l'état de la technique en procurant un moyen de diagnostic plus rapide, plus pratique, plus économique, plus efficace et qui permet une détection et/ou une quantification d'analytes qui est :
- spécifique, c'est-à-dire qui parvient à distinguer des analytes de classes différentes, et
- universelle, c'est-à-dire qui est applicable pour la plupart des substances utiles à analyser dans les domaines de l'agro-alimentaire et du diagnostic médical telles que les résidus médicamenteux (par exemple les antibiotiques et les antibactériens), les toxines, les hormones, les pathogènes, les adultérants ou encore les allergènes,
la détection et/ou la quantification étant réalisée en une seule étape et en moins de 15 minutes.

Plus particulièrement, un moyen de diagnostic selon l'invention permet la détection et/ou la quantification d'au moins 5 classes différentes d'analytes, de préférence d'au moins 10 classes différentes d'analytes, de préférence d'au moins 15 classes différentes d'analytes présents dans un échantillon, les classes d'analytes étant des résidus médicamenteux (par exemple des antibiotiques ou des antibactériens), des toxines, des hormones, des pathogènes, des adultérants ou encore des allergènes, et ce en moins de 15 minutes et en une seule étape. Pour résoudre ce problème, il est prévu suivant l'invention un moyen de diagnostic immuno-chromatographique pour la détection et/ou la quantification respective, simultanée et spécifique d'une pluralité d'analytes présents dans un échantillon essentiellement liquide comprenant :
- au moins un mélange réactionnel contenant des molécules biologiques de reconnaissance et/ou des ligands compétiteurs marqués avec au moins une molécule de visualisation ; et
- au moins un système récupérateur sous la forme d'un support solide auquel se trouvent fixés des ligands compétiteurs et/ou des molécules biologiques de reconnaissance en des emplacements de récupération distincts et connus qui sont disposés selon un arrangement matriciel en deux dimensions, de manière à identifier par la localisation desdits emplacements de récupération sur ledit support, lesdits analytes présents dans ledit échantillon,
ledit moyen de diagnostic étant caractérisé en ce que,
a) ledit arrangement matriciel en deux dimensions est défini selon un système de coordonnées présentant une première coordonnée X et une deuxième coordonnée Y, tel que chaque emplacement de récupération fixé sur ledit support solide permet l'identification d'un analyte distinct et
   avec, pour une même coordonnée X, plusieurs emplacements de récupération chacun comprenant des molécules biologiques de reconnaissance ou des ligands compétiteurs différents, disposés selon des coordonnées Y différentes et, avec, pour une même coordonnée Y, plusieurs emplacements de récupération chacun comprenant des molécules biologiques de reconnaissance ou des ligands compétiteurs différents, disposés selon des coordonnées X différentes ;
b) pour la détection et/ou la quantification d'un analyte donné, un couple de diagnostic constitué d'un ligand compétiteur et d'une molécule biologique de reconnaissance est présent, tel que ladite molécule biologique de reconnaissance se trouve dans ledit mélange réactionnel et ledit ligand compétiteur est fixé en au moins un emplacement de récupération, ou inversement ;
c) ladite au moins une molécule de visualisation est une molécule détectable en fluorescence ;
d) ledit mélange réactionnel est présent dans un contenant, ledit contenant étant distinct et séparé dudit système récupérateur, l'interaction du mélange réactionnel avec l'échantillon à analyser étant focalisée dans ledit contenant distinct dudit système récupérateur de telle sorte que ledit échantillon interagit complètement avec le mélange réactionnel avant que le liquide ainsi obtenu, formé du mélange réactionnel et de l'échantillon, ne soit en contact avec le support solide et donc avec les emplacements de récupération, et
e) ledit système récupérateur comprend au moins 5 emplacements de récupération distincts destinés à la détection et/ou la quantification respective, simultanée et spécifique d'au moins 5 analytes distincts présents dans un échantillon, et au moins un emplacement de récupération destiné à un contrôle et/ou un calibrateur.

Par le terme « analyte », on entend au sens de la présente invention un composé qui constitue un intérêt à être détecté et/ou quantifié pour fournir un diagnostic, particulièrement dans les domaines agro-alimentaire et médical.

Par les termes « classe d'analytes », on entend au sens de la présente invention un groupement de plusieurs analytes qui présentent des propriétés biologiques et chimiques similaires. A titre d'exemple, les résidus médicamenteux peuvent être séparés en différentes classes telles que les pénicillines, les céphalosporines, les tétracyclines, les sulfamides, les aminoglycosides, les aminocyclitols, les macrolides, les quinolones, les ionophores, les carbadox, les nitrofurannes et les phénicols. Particulièrement, les pénicillines sont des antibiotiques qui ont un mode d'action commun (propriété biologique) et qui présentent une structure chimique similaire (propriété chimique).

Par les termes « détection et/ou quantification respective », on entend au sens de la présente invention une détection et/ou une quantification de tous les analytes d'intérêts en utilisant un seul moyen de diagnostic selon l'invention.

Par les termes « détection et/ou quantification simultanée », on entend au sens de la présente invention une détection et/ou une quantification de tous les analytes d'intérêt après un lapse de temps identique.

Par les termes « détection et/ou quantification spécifique », on entend au sens de la présente invention une détection et/ou une quantification de tous les analytes d'intérêt de manière distincte, de telle sorte à pouvoir identifier précisément l'analyte qui est détecté et/ou quantifié.

Par les termes « couple de diagnostic », on entend au sens de la présente invention deux molécules complémentaires destinées à la détection et/ou la quantification d'un analyte donné, lesdites deux molécules étant une molécule de reconnaissance biologique et un ligand compétiteur. La détection et/ou la quantification de l'analyte donné est basé sur le principe de compétition selon deux situations possibles :
- soit la molécule biologique de reconnaissance se trouve dans le mélange réactionnel et le ligand compétiteur complémentaire est fixé sur le support solide ;
- soit le ligand compétiteur se trouve dans le mélange réactionnel et la molécule biologique de reconnaissance complémentaire est fixée sur le support solide.

Par les termes « molécules biologiques de reconnaissance », on entend au sens de la présente invention, une molécule naturelle ou synthétique qui est capable de se lier spécifiquement à un analyte d'intérêt.

Par les termes « ligands compétiteurs », on entend au sens de la présente invention une molécule qui est capable de se lier spécifiquement aux molécules biologiques de reconnaissance et qui va donc entrer en compétition avec **un** analyte d'intérêt pour la liaison aux molécules biologiques de reconnaissance.

Par les termes « emplacement de récupération », on entend au sens de la présente invention **un** emplacement auquel vont être fixés des molécules biologiques de reconnaissance ou des ligands compétiteurs. Dans le cas où des molécules biologiques de reconnaissance sont fixées à un emplacement de récupération, l'analyte d'intérêt (s'il est présent) ou le ligand compétiteur (si l'analyte d'intérêt est absent) va se lier spécifiquement, être capturé et donc arrêter de migrer. Dans le cas où des ligands compétiteurs sont fixés à un emplacement de récupération, les molécules biologiques de reconnaissance spécifiques d'un analyte d'intérêt, vont se lier spécifiquement, être récupérées et donc arrêter de migrer, et ce si l'analyte d'intérêt est absent.

Le procédé mis en œuvre pour la détection et/ou la quantification est le suivant :
- mettre en contact le mélange réactionnel avec l'échantillon pour obtenir un liquide ;
- incuber à une température de 30°C pendant 3 minutes ;
- tremper l'extrémité du système récupérateur qui se trouve en amont du sens de migration dans le liquide (comprenant l'échantillon et le mélange réactionnel) ;
- incuber pendant 10 minutes à 30°C ; et
- interpréter qualitativement et/ou quantitativement le résultat sur le système récupérateur au moyen d'un dispositif de lecture optique.
Le sens de migration du liquide selon l'invention est défini selon ledit système de coordonnées définissant l'arrangement matriciel des emplacements de récupération fixés sur le système récupérateur selon l'invention et se fait par conséquent selon une coordonnée *X* et une coordonnée *Y.*

La détection et/ou la quantification selon l'invention est basée sur le principe de compétition qui exploite la reconnaissance des analytes recherchés vis-à-vis d'un ligand compétiteur et/ou d'une molécule biologique de reconnaissance.

Plusieurs cas peuvent se présenter selon que des ligands compétiteurs ou des molécules biologiques de reconnaissance sont fixés aux emplacements de récupération :
1) Dans le cas où les éléments récupérateurs sont les ligands compétiteurs,
   - soit le composé recherché est présent dans l'échantillon et va alors se lier aux molécules biologiques de reconnaissance présentes dans un mélange réactionnel qui ne seront par conséquent plus libres de se lier aux molécules compétitrices fixées au système récupérateur. Le résultat sera alors positif et présentera un marquage qui est absent ;
   - soit le composé recherché est absent de l'échantillon, les molécules biologiques de reconnaissance présentes dans un mélange réactionnel étant donc libres de se lier aux molécules compétitrices fixées au système récupérateur. Le résultat sera alors négatif et présentera un marquage qui est présent.
2) Dans le cas où les éléments récupérateurs sont les molécules biologiques de reconnaissance,
   - soit le composé recherché est présent dans l'échantillon et va alors entrer en compétition avec les ligands compétiteurs présents dans un mélange réactionnel pour se lier aux molécules biologiques de reconnaissance fixées au système récupérateur. Le résultat sera alors positif et présentera un marquage qui est absent ou faible ;
   - soit le composé recherché est absent de l'échantillon, les ligands compétiteurs étant alors les seuls se lier aux molécules biologiques de reconnaissance fixées au système récupérateur. Le résultat sera alors négatif et présentera un marquage qui est présent.

Dans le cadre de la présente invention, il a été démontré de manière surprenante qu'un moyen de diagnostic immuno-chromatographique qui comprend les caractéristiques (a), (b) (c) et (d) telles qu'indiquées ci-dessus font que le moyen de diagnostic selon l'invention est plus efficace et est à la fois spécifique et à la fois universel. Précisément, il permet de détecter et/ou de quantifier au moins 5 classes différentes d'analytes, de préférence au moins 10 classes différentes d'analytes, de préférence au moins 15 classes différentes d'analytes présents dans un échantillon, les classes d'analytes étant des résidus médicamenteux (par exemple des antibiotiques ou des antibactériens), des toxines, des hormones, des pathogènes, des adultérants ou encore des allergènes, et ce en moins de 15 minutes et en une seule étape. Un moyen de diagnostic selon l'invention est par conséquent plus pratique, plus économique et plus efficace que les moyens de diagnostic connus actuellement qui se limitent à la détection et/ou quantification de moins de cinq classes différentes d'analytes.

Précisément, il a été observé de façon surprenante que le placement du mélange réactionnel dans un contenant séparé du support solide apportait plusieurs avantages.

Premièrement, l'interaction du mélange réactionnel avec l'échantillon a analysé étant focalisée dans un contenant séparé, le contrôle de l'interaction du mélange réactionnel avec l'échantillon est optimisé. De la sorte, il est certain que l'échantillon interagit complètement avec le mélange réactionnel avant que le liquide ainsi obtenu (formé du mélange réactionnel et de l'échantillon) ne soit en contact avec le support solide et donc les emplacements de récupération. Par conséquent, la séparation du mélange réactionnel dans un contenant séparé du support solide permet d'éviter l'obtention de faux négatifs. En effet, dans le cas où le mélange réactionnel est fixé sur le support solide en amont des éléments de récupération par rapport au sens de migration comme c'est le cas dans le document de Taranova *et al*., l'échantillon essentiellement liquide est directement mis en contact avec le mélange réactionnel fixé sur le support solide, ce qui va entrainer la migration immédiate du liquide par capillarité. Le risque est alors élevé que l'échantillon rencontre les emplacements de récupération avant que l'interaction avec le mélange réactionnel ne soit complète entraînant un résultat erroné, c'est-à-dire que l'analyte est effectivement présent dans l'échantillon mais qu'il n'est pas détecté.

Deuxièmement, la séparation du mélange réactionnel dans un contenant permet d'avoir un meilleur contrôle de la quantité d'échantillon qui est analysée. En effet, avec un moyen de diagnostic selon l'invention, il est possible de déposer un volume d'échantillon défini et précis dans le contenant et de s'assurer que la totalité de ce volume d'échantillon va être analysé, et ce contrairement au moyen de diagnostic divulgué par Taranova *et al*.. En effet, avec un tel moyen de diagnostic, c'est-à-dire où le mélange réactionnel est présent sur le support solide en amont des éléments de récupération par rapport au sens de migration du liquide, l'échantillon est directement mis en contact avec le support solide qui est plongé dans l'échantillon, le liquide obtenu (formé de l'échantillon et du mélange réactionnel) migrant instantanément par capillarité. De cette manière, il est impossible de définir précisément le volume de liquide qui va migrer sur le support solide, ce qui rend très difficile voire impossible de déterminer le volume d'échantillon qui est réellement analysé. Cette caractéristique distinctive du moyen de diagnostic selon l'invention permet de réduire les déviations standards et d'obtenir ainsi une reproductibilité améliorée par rapport aux moyens de diagnostic de l'état de l'art. La détermination précise du volume d'échantillon qui est analysé permet également de définir de manière plus adéquate la composition du mélange réactionnel et surtout de la quantité des différents éléments qui le composent. Par conséquent, la détection ou la quantification d'un analyte donné est significative et fiable même en simplicat, et ce contrairement au document de Taranova. En effet, selon ce document antérieur et comme cité plus haut, huit emplacements de récupération doivent être prévus sur le support solide pour la détection et la quantification d'un seul analyte. Donc, pour la détection et/ou la quantification fiable d'un même nombre d'analytes, par exemple quatre analytes, un support solide selon l'invention doit comprendre 4 emplacements de récupération, alors qu'un support solide selon Taranova *et al.* doit comprendre 32 emplacements de récupération. Par conséquent, pour une même surface de support solide, le moyen de diagnostic selon l'invention permet de détecter et/ou de quantifier 32 analytes différents.

Troisièmement, la séparation du mélange réactionnel dans un contenant permet d'augmenter le nombre de constituants du mélange réactionnel. En effet, comme décrit ci-dessus, pour la détection et/ou la quantification d'un analyte donné, un couple de diagnostic constitué d'un ligand compétiteur et d'une molécule biologique de reconnaissance est présent, tel que la molécule biologique de reconnaissance se trouve dans le mélange réactionnel et le ligand compétiteur est fixé en au moins un emplacement de récupération, ou inversement. Donc, le mélange réactionnel contient une molécule de reconnaissance ou un ligand compétiteur par analyte. Par conséquent, pour détecter et/ou quantifier un grand nombre d'analytes différents, un plus grand nombre de molécules de reconnaissance ou de ligands compétiteurs différents devra être ajouté dans le mélange réactionnel. Avec les moyens de diagnostic de l'état de la technique, comme divulgué par Taranova *et al*., le nombre de constituants du mélange réactionnel est limité par la surface disponible sur le support solide.

En outre, selon l'invention, l'arrangement matriciel en deux dimensions est défini selon un système de coordonnées présentant une première coordonnée *X* et une deuxième coordonnée *Y,* tel que chaque emplacement de récupération fixé sur ledit support solide permet l'identification d'un analyte distinct. Cette caractéristique améliore également significativement l'efficacité du moyen de diagnostic selon l'invention en procurant un moyen de diagnostic qui permet de détecter et/ou de quantifier un nombre plus élevé d'analytes distincts pour une surface de support identique, par exemple d'améliorer l'efficacité de huit fois par rapport au moyen de diagnostic selon Taranova *et al*..

Ainsi, selon l'invention, pour une même coordonnée *X,* plusieurs emplacements de récupération, chacun comprenant des molécules biologiques de reconnaissance ou des ligands compétiteurs différents, sont disposés selon des coordonnées *Y* différentes permettant ainsi de détecter et/ou de quantifier, pour une même coordonnée *X,* plusieurs analytes différents. Inversement, pour une même coordonnée *Y,* plusieurs emplacement de récupérations, chacun comprenant des molécules biologiques de reconnaissance ou des ligands compétiteurs différents, sont disposés selon des coordonnées *X* différentes, permettant ainsi de détecter et/ou de quantifier plusieurs analytes différents.

Par ailleurs, il a été observé de manière surprenante que l'utilisation d'une molécule de visualisation qui est détectable en fluorescence permet d'améliorer la limite de détection du signal, et donc de réduire le risque de faux négatifs en augmentant la sensibilité de la détection et/ou de la quantification. Par conséquent, le seuil de détection étant plus bas, les emplacements de récupération peuvent être plus petits, ce qui permet d'obtenir un support solide qui comprend plus d'emplacements de récupération pour une surface identique. Par ailleurs, la détection du signal par fluorescence étant plus sensible, une quantité plus faible de ligands compétiteurs et/ou de molécules biologiques de reconnaissance doivent être fixés aux emplacements de récupération, ce qui donne un avantage économique non négligeable, mais également un bruit de fond qui est réduit et un risque d'inter-réactions entre les mécanismes de détection et/ou quantification qui est diminué.

En conclusion, un moyen de diagnostic selon l'invention fournit un effet technique supérieur par rapport aux moyens de diagnostic actuels et plus particulièrement par rapport au moyen de diagnostic selon le document de Taranova *et al*..

Ainsi, la présente invention démontre la compatibilité technique et pratique de réunir en un seul et même moyen de détection un nombre élevé (au moins 5, de préférence au moins 10, de préférence au moins 15) de mécanismes de détection et/ou de quantification. Il a par ailleurs été mis en avant, dans le cadre de la présente invention, une faisabilité technique d'un dosage multi-analytes qui peut s'opérer rapidement, en moins de 15 minutes, préférentiellement en 13 minutes, et en une seule et même étape d'analyse à l'aide d'un seul et même échantillon. En effet, le moyen de diagnostic selon l'invention ne requiert ni de lavages ni la réalisation d'une étape distincte de marquage des molécules de reconnaissance et/ou des ligands compétiteurs avec au moins une molécule de visualisation étant donné que, selon l'invention, le mélange réactionnel comprend des molécules de reconnaissance et/ou des ligands compétiteurs couplés à au moins une molécule de visualisation. De préférence, lesdits emplacements de récupération fixés sur ledit système récupérateur dudit moyen de diagnostic selon l'invention sont disposés selon un arrangement matriciel en deux dimensions sous la forme de points présentant chacun un diamètre compris entre 20 µm et 2 mm, de préférence compris entre 100 µm et 500 µm, préférentiellement entre 250 µm et 400 µm.

Il a été démontré que des emplacements de récupération sous forme de points présentant chacun un diamètre compris entre 20 µm et 2 mm, de préférence compris entre 100 µm et 500 µm, préférentiellement entre 250 µm et 400 µm, permettaient de fixer au moins 5, de préférence au moins 10, préférentiellement au moins 15 emplacements de récupération en simplicat, en duplicat ou en triplicat pour la détection et/ou la quantification d'au moins 5, de préférence au moins 10, préférentiellement au moins 15 analytes différents, ainsi qu'au moins un emplacement de récupération déposé en simplicat, en duplicat ou en triplicat destiné au contrôle du seuil de détection permettant de valider le test et/ou à la calibration pour la détection et/ou la quantification, et ce sur un système récupérateur ayant une taille raisonnable, pour la réalisation de la détection et/ou la quantification desdits au moins 15 analytes par un dispositif de lecture optique.

Avantageusement, les emplacements de récupération fixés sur ledit système récupérateur dudit moyen de diagnostic selon l'invention sont disposés selon un arrangement matriciel en deux dimensions sous la forme de points, lesdits points étant présents à une densité comprise entre 62500 et 6,25 points par cm², de préférence comprise entre 2500 et 100 points par cm², préférentiellement comprise entre 400 et 150 points par cm².

De préférence, l'arrangement matriciel de tous les emplacements de récupération est inférieur ou égal à 3 cm², de préférence inférieure ou égale à 2 cm², de préférence inférieure ou égale à 1 cm².

Avantageusement, la première coordonnée *X* est définie sur un axe longitudinal d'une longueur dudit système récupérateur et la deuxième coordonnées *Y* est définie sur un axe longitudinal d'une largeur dudit système récupérateur.

Il est raisonnable de prévoir une distance d'espacement minimal entre deux points qui est comprise entre 20 µm et 2 mm, de préférence entre 100 µm et 500 µm, préférentiellement entre 250 µm et 400 µm, selon des coordonnées *X* et *Y.*

Le système récupérateur selon l'invention comprend au moins 5, de préférence au moins 10, préférentiellement au moins 15 emplacements de récupération distincts destinés la détection et/ou la quantification respective, simultanée et spécifique d'au moins 5, de préférence au moins 10, préférentiellement au moins 15 analytes distincts présents dans un échantillon, et au moins un emplacement de récupération destiné à un contrôle et/ou un calibrateur.

De préférence, ledit contrôle et/ou ledit calibrateur est obtenu à partir d'un couple ligand compétiteur/molécule de reconnaissance indépendant, dont la nature intrinsèque (ou synthétique) fait que la molécule contrôle n'est jamais présente dans l'échantillon (par exemple un anticorps spécifique d'une protéine d'une autre espèce animale différente de celle dont provient l'échantillon) ou une protéine porteuse (par exemple la sérumalbumine bovine) modifiée chimiquement avec un marqueur synthétique (par exemple une biotine ou un marqueur poly-histidines ou c-myc).

Dans une forme de réalisation particulièrement avantageuse du moyen de diagnostic selon l'invention, chacun desdits emplacements de récupération est disposé sur ledit système récupérateur en duplicat, de préférence en triplicat. La réalisation de duplicats ou de triplicats permet d'améliorer encore les statistiques et la précision des résultats obtenus.

De préférence, l'arrangement matriciel des emplacements de récupération auxquels sont fixés des ligands compétiteurs ou les molécules de reconnaissance est déterminé par le sens de migration du liquide, de telle sorte qu'un emplacement de récupération auquel sont fixés des ligands compétiteurs ou des molécules biologiques de reconnaissance destinés à la détection et/ou la quantification d'un premier analyte donné est localisé en amont d'un emplacement de récupération auquel sont fixés des ligands compétiteurs ou des molécules biologiques de reconnaissance destinés à la détection et/ou la quantification d'un second analyte donné, et ce par rapport au sens de migration du liquide. Un tel arrangement matriciel permet encore de diminuer le risque d'inter-réactions entre les différents mécanismes de détection et/ou de quantification des analytes d'intérêt.

Avantageusement, ledit système récupérateur sous la forme d'un support solide comprend une membrane ou un ensemble de membranes. De préférence, la membrane est une membrane de nitrocellulose.

Avantageusement, ledit contenant est un contenant en verre ou en plastique.

Avantageusement, lesdites molécules biologiques de reconnaissance sont des anticorps, de préférence des anticorps primaires, soit monoclonaux ou polyclonaux, purifiés ou non purifiés, et/ou des aptamères et/ou des GEPIs et/ou des récepteurs biologiques.

Avantageusement, lesdits ligands compétiteurs sont des analogues des analytes recherchés et/ou des molécules capables de fixer spécifiquement lesdites molécules biologiques de reconnaissance.

Dans une forme de réalisation particulièrement avantageuse du moyen de diagnostic selon l'invention, lesdits ligands compétiteurs sont choisis dans le groupe constitué de substances médicamenteuses de type antibiotiques, d'hormones, de toxines telle que l'Aflatoxine, de virus de type virus de Dengue, de bactéries de type L. monocytogenes, de métaux lourds, d'adultérants, d'allergènes, et de leurs mélanges.

De préférence, ladite au moins une molécule de visualisation est fusionnée auxdites molécules biologiques de reconnaissance et/ou auxdits ligands compétiteurs via un couplage chimique et/ou génétique.

Par les termes « couplage chimique et/ou génétique », il est entendu au sens de la présente invention, une liaison de la molécule de reconnaissance et/ou du ligand compétiteur à la molécule de visualisation via une modification chimique et/ou génétique de la molécule biologique de reconnaissance et/ou du ligand compétiteur, ceux-ci n'étant par conséquent plus dans leur état naturel mais sous forme modifiée ou sous forme de complexes.

Il a été démontré qu'un tel couplage chimique et/ou génétique de la molécule de visualisation aux molécules biologiques de reconnaissance et/ou aux ligands compétiteurs présents dans le mélange réactionnel permet d'améliorer encore la compatibilité technique et pratique de réunir en un seul et même moyen de détection et/ou de quantification un nombre élevé (au moins 5, de préférence au moins 10, préférentiellement au moins 15) de mécanismes de détection et/ou de quantification sans que le fonctionnement de l'un d'entre eux ne puisse interférer avec le fonctionnement d'un des autres mécanismes. En effet, un mélange réactionnel selon l'invention qui présente un tel couplage offre l'avantage de diminuer voir d'éliminer le risque d'aspécificité et d'interréactions entre les différentes molécules biologiques de reconnaissance et/ou les différents ligands compétiteurs présents dans le mélange réactionnel, et ainsi le risque d'observer des faux positifs et/ou des faux négatifs, mais également de diminuer considérablement le marquage résiduel (bruit de fond) observé sur le système récupérateur quand un tel couplage n'est pas présent et d'obtenir ainsi un meilleur contraste entre le marquage des éléments récupérateurs et du support solide non fixé (et d'obtenir ainsi un meilleur seuil de détection).

Le document antérieur EP1712914 préconise, au contraire, qu'aucun marquage par modification chimique n'ait lieu afin de préserver au maximum les fonctionnalités des récepteurs et anticorps utilisés, et que par conséquent, les molécules biologiques de reconnaissance sont exploitées dans leur état le plus naturel possible. Par exemple, une molécule biologique de reconnaissance comme un récepteur est marqué à l'aide d'un anticorps eux-mêmes reconnus par une protéine-A (reconnaissant tous les types d'anticorps de manière générale) qui est conjuguée à de l'or colloïdal. Selon ce document antérieur, c'est donc la protéine-A, et non la molécule biologique de reconnaissance, qui est couplée à l'or colloïdal (la molécule de visualisation).

Avantageusement, ledit couplage chimique et/ou génétique est réalisé via au moins une force électrostatique, au moins un lien peptidique, au moins un gène rapporteur, ou une combinaison de ceux-ci.

Dans une forme de réalisation particulière, ladite au moins une molécule de visualisation est choisie dans le groupe constitué de l'isothiocyanate de fluorescéine (FITC), la phycoérythrine (PE), la rhodamine B et de leurs mélanges.

De préférence, lesdits analytes sont sélectionnés dans le groupe constitué des résidus médicamenteux, des toxines, des virus, des bactéries, des hormones, des métaux lourds, des adultérants ,es allergènes et de leurs mélanges. Parmi les résidus médicamenteux, on retrouve notamment les antibiotiques et les antibactériens. Des molécules chimiques indésirables, les adultérants, peuvent également être détectées suite à une contamination passive par transfert du contenant (par exemple depuis un emballage en plastique).

Dans une forme de réalisation particulièrement avantageuse, lesdits analytes sont des résidus médicamenteux et sont choisis dans le groupe constitué des pénicillines, des céphalosporines, des tétracyclines, des sulfamides, des aminoglycosides, des aminocyclitols, des macrolides, des quinolones, des ionophores, des carbadox, des nitrofurannes, des phénicols, et de leurs mélanges.

Avantageusement, ledit échantillon est obtenu à partir de lait, de miel, de viande, d'œufs, de sang complet, de sérum, d'urine, ou d'autres liquides biologiques.

Par les termes « liquides biologiques », on entend au sens de la présente invention tout liquide organique ou fluide corporel produit par un organisme vivant.

De préférence, ledit échantillon est obtenu à partir de lait. Il a été observé que la détection des analytes est plus sensible lorsque l'échantillon analysé est obtenu à partir de lait, et ce car les composants du lait sature la membrane de nitrocellulose et diminue ainsi le bruit de fond.

Particulièrement, selon l'invention, la détection et/ou la quantification respective, simultanée et spécifique d'une pluralité d'analytes présents dans un échantillon est réalisée au moyen d'un dispositif de lecture optique.

D'autres formes de réalisation du moyen de diagnostic suivant l'invention sont indiquées dans les revendications annexées.

L'invention porte également sur un procédé pour la détection et/ou la quantification respective, simultanée et spécifique d'une pluralité d'analytes présents dans un échantillon essentiellement liquide comprenant les étapes suivantes :
- mettre en contact un mélange réactionnel d'un moyen d'un diagnostic selon l'invention avec l'échantillon pour obtenir un liquide ;
- incuber à une température comprise entre 0 et 70°C, de préférence entre 10 et 60°C, de préférence entre 20 et 50°C, de préférence entre 20 et 40°C, de préférence entre 25 et 35°C, de préférence de 30°C, pendant une durée inférieure ou égale à 15 minutes, préférentiellement inférieure ou égale à 10 minutes, préférentiellement inférieure ou égale à 5 minutes, préférentiellement inférieure ou égale à 3 minutes, préférentiellement égale à 3 minutes ;
- tremper une extrémité d'un système récupérateur d'un moyen de diagnostic selon l'invention dans le liquide ;
- incuber à une température comprise entre 0 et 70°C, de préférence entre 10 et 60°C, de préférence entre 20 et 50°C, de préférence entre 20 et 40°C, de préférence entre 25 et 35°C, de préférence de 30°C, pendant une durée inférieure ou égale à 15 minutes, préférentiellement inférieure ou égale à 10 minutes, préférentiellement égale à 10 minutes ; et
- interpréter qualitativement et/ou quantitativement le résultat sur le système récupérateur au moyen d'un dispositif optique.

Le procédé selon l'invention est basé sur les technologies de la microfluidique et de l'immuno-chromatographie.

L'invention a aussi pour objet un ensemble de diagnostic pour la détection et/ou la quantification respective, simultanée et spécifique d'analytes présents dans un échantillon comprenant un moyen de diagnostic selon l'invention, et comprend en outre un dispositif de lecture optique d'un support solide amovible comprenant :
- un emplacement pour recevoir ledit support solide ;
- une unité optique pour analyser ledit support solide et comprenant :
   ∘ une première source lumineuse pour émettre selon une intensité d'émission et dans une première plage de longueur d'onde un premier faisceau lumineux vers ledit emplacement ;
   o un système d'imagerie comprenant un détecteur optique pour fournir une image d'une zone de visualisation, ladite zone de visualisation comprenant au moins une portion dudit emplacement ;
   o un filtre pour filtrer une plage de longueur d'onde définie, et positionné entre l'emplacement et ledit système d'imagerie ;
- des moyens de communications pour obtenir une information relative à un support solide ;
- des moyens de sélection pour :
   o sélectionner à partir d'une liste d'analytes prédéfinis correspondant auxdits emplacements de récupération fixés sur le support solide, une sélection d'analytes à détecter et/ou à quantifier pour ledit échantillon à partir d'un même support solide ;
- des moyens de traitement d'image de ladite image pour :
   ∘ déterminer, à partir de l'information relative audit support solide à lire, un nombre fini de sous-ensembles de ladite image, chaque sous-ensemble correspondant à un analyte ;
   o fournir des données relatives à des intensités lumineuses issues desdits sous-ensembles;
- des moyens de détermination pour:
   o calculer, pour chaque sous-ensemble correspondant à un analyte sélectionné dans ladite sélection d'analytes une intensité de sous-ensemble ;
   o déterminer sur la base de ladite intensité de sous-ensemble des informations d'analytes dudit échantillon pour chaque sous-ensemble correspondant à un analyte sélectionné dans ladite sélection d'analytes ;
- des moyens de transmission configurés pour transmettre ladite information d'analytes dudit échantillon analysé pour chaque sous-ensemble correspondant à un analyte sélectionné dans ladite sélection d'analytes.

Un tel dispositif selon l'invention permet la lecture des zones à tester, en particulier avec une mesure instantanée de fluorescence ou de façon préférée avec une mesure de la lumière réfléchie des zones à tester. Afin de permettre une sélection des analytes à tester correspondant à des zones d'intérêts sur une tigette, un tel dispositif propose grâce à l'accès à une méthode contenant des informations relatives à une tigette, une sélection à partir d'une liste d'analytes à tester. Il est en effet intéressant d'effectuer une sélection des analytes à tester avant d'en obtenir les résultats afin de bien cibler les analytes dont il est nécessaire de connaitre les résultats d'un test afin de ne pas exposer un utilisateur à une trop grande quantité de résultats. Donner accès à une trop grande quantité de résultats à un utilisateur n'en ayant pas nécessairement le besoin l'expose au risque d'une perte d'objectivité par rapport à son intention d'analyse initiale. Ainsi, un tel dispositif de l'invention, grâce à des moyens de sélection, permet un choix des analytes à tester par l'utilisateur avant d'effectuer la lecture de la tigette. Les moyens de sélection, en communication avec les moyens de traitement d'image permettent aux moyens de traitement d'image, une détermination des informations des analytes sélectionnés uniquement.

Un avantage d'utiliser le lecteur optique de l'invention pour réaliser un diagnostic concernant une sélection d'analytes d'intérêt est qu'il ne nécessite ni une première sélection de différents types de bandelettes à tester, ni la mise en contact de chacune de ces bandelettes avec le produit à tester puis leur positionnement dans le lecteur optique. Tout cela permet d'éviter une manipulation importante des bandelettes à tester, coûteuse en temps et en gestion des stocks. Cela permet également une analyse plus simple, plus rapide et plus ciblée des analytes à tester, en disposant uniquement des résultats sélectionnés à l'issue de la lecture de la bandelette par le lecteur optique de l'invention.

Avantageusement, la sélection d'analytes est une sélection de plusieurs analytes.

De préférence, le dispositif optique comprend en outre :
- des moyens permettant de lire **un** profil de sélection ; et
lesdits moyens de sélection sont configurés pour réaliser ladite sélection d'analytes sur la base dudit profil de sélection.

De manière avantageuse, chaque sous-ensemble dudit nombre fini de sous-ensembles de ladite image déterminé par les moyens de traitement d'image correspond à ladite sélection d'analytes, de préférence à chaque analyte sélectionné.

Préférentiellement, lesdits moyens de traitement d'image sont configurés pour déterminer en outre ledit nombre fini de sous-ensembles de ladite image à partir dudit profil de sélection.

Avantageusement, ladite première source lumineuse est configurée pour émettre directement ledit premier faisceau lumineux directement vers ledit emplacement, de préférence directement vers lesdits emplacements de récupération fixés sur le support solide.

De préférence, ledit support solide comprend des emplacements de récupération sous forme de points présentant chacun **un** diamètre compris entre 20 µm et 2 mm, de préférence compris entre 100 µm et 500 µm, préférentiellement entre 250 µm et 400 µm.

L'invention porte également sur un ensemble de diagnostic pour la détection et/ou la quantification respective, simultanée et spécifique d'analytes présents dans un échantillon comprenant un moyen de diagnostic selon l'invention et comprend en outre un dispositif de lecture optique d'un support solide amovible comprenant :
- un emplacement pour recevoir ledit support solide ;
- une unité optique pour analyser ledit support solide et comprenant :
   o une première source lumineuse pour émettre selon une intensité d'émission et dans une première plage de longueur d'onde **un** premier faisceau lumineux vers ledit emplacement ;
   o un capteur d'intensité lumineuse pour mesurer l'intensité d'émission émise par ladite première source lumineuse ;
   o un moyen de rétrocontrôle pour moduler ladite intensité d'émission de ladite première source lumineuse en fonction de l'intensité d'émission mesurée par ledit capteur d'intensité lumineuse de sorte que ladite première source lumineuse émette une intensité cible ;
   o un système d'imagerie comprenant un détecteur optique pour fournir une image d'une zone de visualisation, ladite zone de visualisation comprenant au moins une portion dudit emplacement ;
   o un filtre pour filtrer une plage de longueur d'onde définie, et positionné entre l'emplacement et ledit système d'imagerie ;
- des moyens de traitement d'image de ladite image pour :
   o déterminer un nombre fini de sous-ensembles de ladite image,
   o fournir des données relatives à des intensités lumineuses issues desdits sous-ensembles ;
- des moyens de détermination pour:
   o calculer, pour chaque sous-ensemble, une intensité de sous-ensemble, et
- des moyens de transmission pour transmettre une information relative à ladite intensité de sous-ensemble pour chaque sous-ensemble.

Un tel dispositif optique selon l'invention permet la lecture des zones à tester, en particulier avec une mesure instantanée de fluorescence ou de façon préférée avec une mesure de la lumière réfléchie des zones à tester. Lorsqu'une technique de fluorescence ou une technique en lumière réfléchie est utilisée pour lire les zones à tester (ou points), un moyen de rétrocontrôle permet de garantir une intensité lumineuse d'excitation toujours égale, ce qui permet une mesure instantanée de fluorescence ou de réflexion fiable, quelle que soit la température, la source d'énergie utilisée ou encore la durée d'utilisation et le vieillissement de la source lumineuse. L'utilisation du moyen de rétrocontrôle permet de garantir une source d'énergie lumineuse ayant une intensité constante dans le temps et prédéfinie. Une source d'énergie lumineuse ayant une intensité prédéfinie permet notamment de garantir des résultats quantitatifs fiables. Le moyen de rétrocontrôle est de préférence un moyen de rétrocontrôle électronique. Par exemple le capteur d'intensité lumineuse est une photodiode.

L'invention a également pour objet un ensemble de diagnostic pour la détection et/ou la quantification respective, simultanée et spécifique d'analytes présents dans un échantillon comprenant un moyen de diagnostic selon l'invention et comprend en outre un dispositif de lecture optique d'un support solide amovible comprenant :
- un emplacement pour recevoir ledit support solide ;
- une unité optique pour analyser ledit support solide et comprenant :
   ∘ une première source lumineuse pour émettre selon une intensité d'émission et dans une première plage de longueur d'onde un premier faisceau lumineux vers ledit emplacement ;
   o un système d'imagerie comprenant un détecteur optique à deux dimensions pour fournir une image à deux dimensions d'une zone de visualisation, ladite zone de visualisation comprenant au moins une portion dudit emplacement ;
   o un filtre pour filtrer une plage de longueur d'onde définie, et positionné entre l'emplacement et ledit système d'imagerie ;
- des moyens de traitement d'image de ladite image à deux dimensions pour :
   ∘ détecter des zones de références de ladite image à deux dimensions,
   ∘ déterminer un nombre fini de sous-ensembles de ladite image à deux dimensions,
   o positionner dans ladite image à deux dimensions chaque sous-ensemble à une position prédéterminée par rapport auxdites zones de référence,
   o fournir des données relatives à des intensités lumineuses issues desdits sous-ensembles ;
- des moyens de détermination pour:
   o calculer, pour chaque sous-ensemble, une intensité de sous-ensemble, et
- des moyens de transmission configurés pour transmettre ladite intensité de sous-ensemble pour chaque sous-ensemble.

Un tel dispositif optique selon l'invention permet la lecture simultanée d'un grand nombre de dots grâce à un détecteur optique à deux dimensions et à des moyens de traitement d'image et de détermination permettant de lire des informations d'analytes pour chacun des dots. L'image à deux dimensions comprend des sous-ensembles, des portions, des régions d'intérêt, des zones d'intérêt ou encore des parties d'images. Préférentiellement, les sous-ensembles d'une image à deux dimensions comprennent une pluralité de pixels. De préférence, chaque sous-ensemble comprend au moins 20 pixels, de préférence plus de 50 pixels et de manière encore plus préférée plus de 200 pixels.

L'avantage d'un tel dispositif selon l'invention est de pouvoir réaliser un diagnostic par une lecture de fluorescence en continu en s'affranchissant un maximum du bruit de fond engendré par la source lumineuse.

Un autre avantage d'un tel dispositif de lecture optique de l'invention est de permettre la lecture optique d'un grand nombre de régions d'intérêt présents sur une seule et même tigette. Dans le cas d'un tel dispositif optique de l'invention, la lecture d'un grand nombre de régions d'intérêt ne nécessite pas de prévoir des emplacements pour plusieurs tigettes. L'emploi de plusieurs tigettes dans un même lecteur optique pour une lecture simultanée de plusieurs tigettes afin de couvrir un grand nombre de régions d'intérêt avec un même capteur optique étant source de mauvais placement et de décalage des régions d'intérêt d'une mesure à une autre et ce pour chacune des tigettes introduites dans le lecteur optique.

L'invention porte également sur un ensemble de diagnostic pour la détection et/ou la quantification respective, simultanée et spécifique d'analytes présents dans un échantillon comprenant un moyen de diagnostic selon l'invention et comprenant en outre_un dispositif de lecture optique d'un support solide amovible comprenant :
- un emplacement pour recevoir ledit support solide ;
- un dispositif d'identification pour identifier un support solide à lire ;
- des moyens de communication pour accéder à une base de données de méthodes relatives audit support solide à lire pour obtenir une information relative à un supporte solide ;
- une unité optique, pour analyser ledit support solide sur la base de paramètres d'analyse compris dans ladite information relative à un support solide et comprenant :
   o une première source lumineuse pour émettre selon une intensité d'émission et dans une première plage de longueur d'onde un premier faisceau lumineux vers ledit emplacement ;
   o un système d'imagerie comprenant un détecteur optique pour fournir une image d'une zone de visualisation, ladite zone de visualisation comprenant au moins une portion dudit emplacement ;
   o un filtre pour filtrer une plage de longueur d'onde définie, et positionné entre l'emplacement et ledit système d'imagerie ;
- des moyens de traitement d'image de ladite image pour :
   o lire dans l'information relative audit support solide à lire, une information relative à un nombre fini de sous-ensembles de ladite image ;
   o fournir des données relatives à des intensités lumineuses issues desdits sous-ensembles ;
- des moyens de détermination pour:
   o calculer, pour chaque sous-ensemble, une intensité de sous-ensemble, et
   o déterminer sur la base de ladite intensité de sous-ensemble et sur la base de l'information relative audit support solide à lire, des informations d'analyte pour chaque sous-ensemble ;
- des moyens de transmission configurés pour transmettre ladite information d'analyte pour chaque sous-ensemble.

Le dispositif de lecture optique selon l'invention permet une lecture optique d'une tigette pour l'analyse d'un échantillon avec un choix de méthode de lecture automatisé. La méthode de lecture comprenant de préférence des données relatives a : une version de méthode, un numéro de lot, une date limite d'utilisation d'un lot, le type de source lumineuse utilisée, le type de zone d'intérêt (ligne ou point), méthode pour analyse qualitative (binaire) ou quantitative, des paramètres d'acquisition d'image (temps d'exposition, gain,...), les positions par rapport à des points de référence (par exemple selon des coordonnées cartésiennes), un nombre de zones d'intérêt, un nombre de réplica par analyte, l'organisation matricielle des zones d'intérêt sur le support solide mobile, la dimensions des zones d'intérêt (par exemple, un rayon), une dimension relative à une zone autour d'une zone d'intérêt à considérer pour la prise en compte du background, des paramètres de calibration du type interpolation de données ou permettant une analyse quantitative d'un échantillon et enfin la désignation des zones d'intérêt en fonction de l'analyte qu'elles permettent de détecter et/ou de quantifier.

D'autres formes de réalisation de l'ensemble de diagnostic suivant l'invention sont indiquées dans les revendications annexées.

L'invention a aussi pour objet une utilisation d'un moyen de diagnostic selon l'invention pour la détection et/ou la quantification respective, simultanée et spécifique d'analytes présents dans un échantillon, d'au moins 5, de préférence d'au moins 10, préférentiellement d'au moins 15 analytes différents.

L'invention a aussi pour objet une utilisation d'un ensemble de diagnostic selon l'invention, pour la détection et/ou la quantification respective, simultanée et spécifique d'analytes présents dans un échantillon, d'au moins 5, de préférence d'au moins 10, préférentiellement d'au moins 15 analytes différents.

D'autres formes d'utilisations du moyen de diagnostic et de l'ensemble de diagnostic suivant l'invention sont indiquées dans les revendications annexées.

D'autres caractéristiques, détails et avantages de l'invention ressortiront de la description donnée ci-après, à titre non limitatif et en faisant référence aux dessins annexés.

### Description des figures

La figure 1a est une vue schématique d'un moyen de diagnostic selon le document antérieur EP1712914.
La figure 1b est une vue schématique d'un moyen de diagnostic selon le document de Taranova *et al*..
La figure 2 est une vue schématique d'un moyen de diagnostic selon l'invention.
La figure 3 est une vue schématique illustrant en détail un système récupérateur selon l'invention.

Sur les figures, les éléments identiques ou analogues portent les mêmes références.

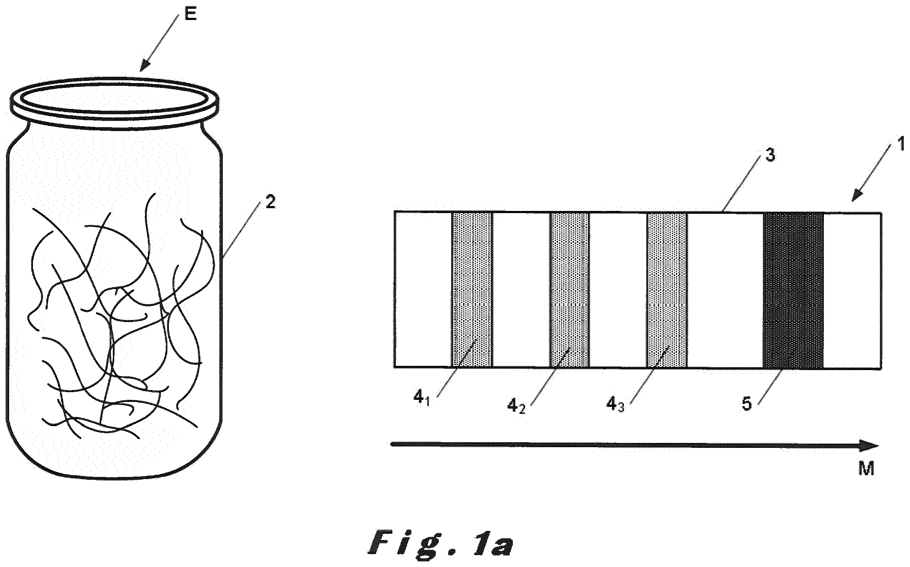

La figure 1a représente un moyen de diagnostic 1 selon le document antérieur EP1712914 et illustre le positionnement des éléments récupérateurs 4₁, 4₂, 4₃ et 5 sur un système récupérateur 3 sous la forme d'un support solide de nitrocellulose dans le cas du dosage simultané de β-lactames 4₁, de tétracyclines 4₂ et de sulphadiméthoxine 4₃, une zone de contrôle fixe 5 étant également prévue, par rapport à un sens de migration M. Selon ce document antérieur, le mélange réactionnel 2 est prévu dans un récipient distinct avec lequel un échantillon E à tester est mis en contact.

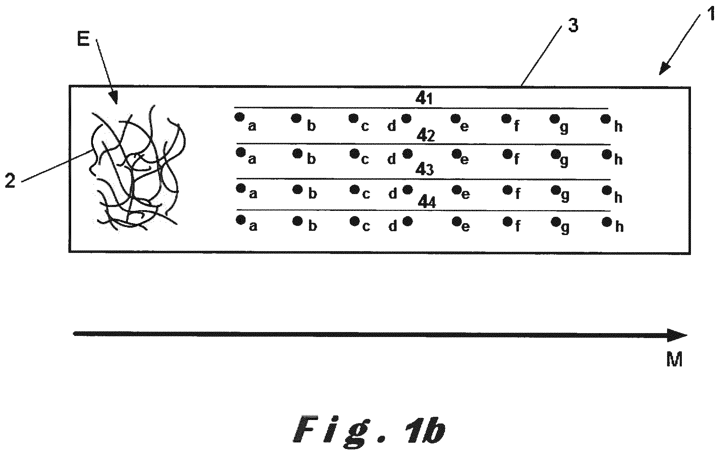

La figure 1b représente un moyen de diagnostic 1 selon le document de Taranova *et al.* et illustre le positionnement des éléments récupérateurs 4₁ₐ, 4_{1b}, 4_{1c}, 4_{1d}, 4₁ₑ, 4_{1f}, 4_{1g}, 4₁ₕ, 4₂ₐ, 4_{2b}, 4_{2c}, 4_{2d}, 4_{2c}, 4_{2f}, 4_{2g}, 4₂ₕ, 4₃ₐ, 4_{3b}, 4_{3c}, 4_{3d}, 4₃ₑ, 4_{3f}, 4_{3g}, 4₃ₕ, 4₄ₐ, 4_{4b}, 4_{4c}, 4_{4d}, 4₄ₑ, 4_{4f}, 4_{4g}, 4₄ₕ, sur un système récupérateur 3 sous la forme d'un support solide de nitrocellulose dans le cas du dosage simultané d'amphétamines (4₁ₐ, 4_{1b}, 4_{1c}, 4_{1d}, 4₁ₑ, 4_{1f}, 4_{1g}, 4₁ₕ), de benzoylecgomine (4₂ₐ, 4_{2b}, 4_{2c}, 4_{2d}, 4_{2c}, 4_{2f}, 4_{2g}, 4₂ₕ ), de méthamphétamines (4₃ₐ, 4_{3b}, 4_{3c}, 4_{3d}, 4_{3c}, 4_{3f}, 4_{3g}, 4₃ₕ) et de morphine (4₄ₐ, 4_{4b}, 4_{4c}, 4_{4d}, 4₄ₑ, 4_{4f}, 4_{4g}, 4₄ₕ). Les éléments récupérateurs 4 sont fixés sous la forme de points selon un arrangement matriciel en deux dimensions. Selon Taranova *et al*., le mélange réactionnel 2 est présent sur ledit système récupérateur 3, sous une forme lyophilisée, en amont desdits éléments de récupération 4 fixés sur ledit système récupérateur 3 par rapport à un sens de migration M d'un liquide comprenant l'échantillon E à tester sur le mélange réactionnel 2. Selon ce document antérieur, les éléments de récupérations disposés sur une même rangée, à savoir ayant la même coordonnée Y, sont spécifiques du même analyte.

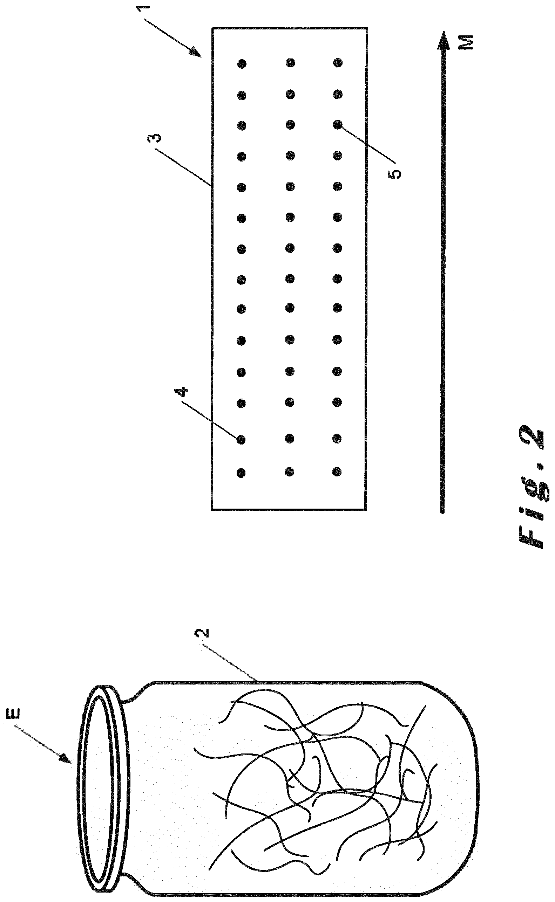

La figure 2 représente un moyen de diagnostic 1 selon l'invention et illustre le positionnement des éléments récupérateurs 4 et 5 sur un système récupérateur 3 sous la forme d'un support solide par rapport à un sens de migration M, les éléments récupérateurs 4 et 5 étant fixés sous la forme de points selon un arrangement matriciel en deux dimensions. Selon l'invention, le mélange réactionnel 2 est prévu dans un récipient distinct avec lequel un échantillon E à tester est mis en contact pour obtenir un liquide, avant de tremper le système récupérateur 3 dans le liquide obtenu.

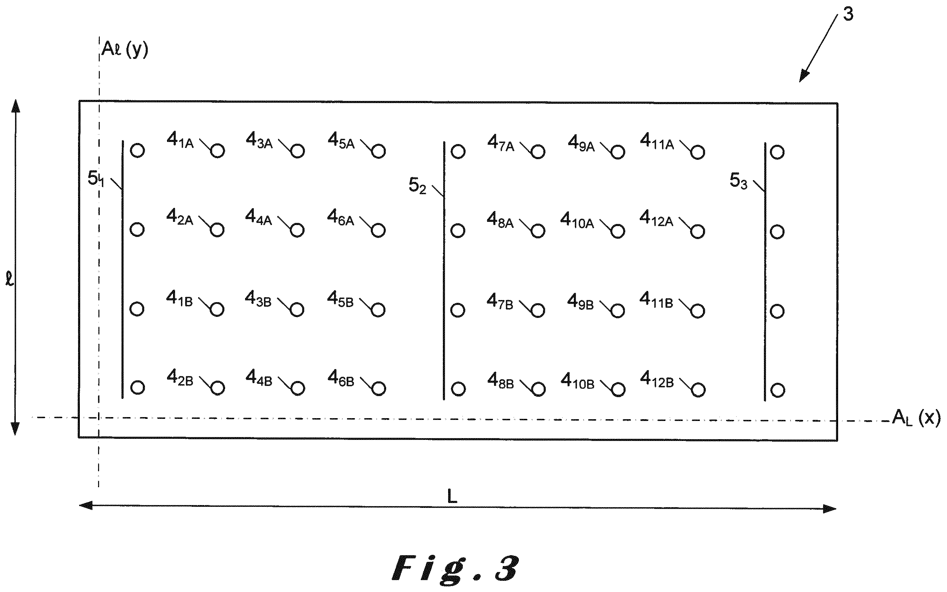

La figure 3 illustre en détail le système récupérateur 3 selon l'invention sur lequel les emplacements de récupération 4 et 5 sont disposés selon un arrangement matriciel en deux dimensions sous la forme de points ayant un diamètre défini, chacun des points étant séparés par une distance minimale. L'arrangement matriciel en deux dimensions est défini selon un système de coordonnées (*X*; Y) qui présente une première coordonnée X définie sur un axe longitudinal (A_{L}) d'une longueur (L) dudit système récupérateur 3 et une deuxième coordonnées Y définie sur un axe longitudinal (A_{I}) d'une largeur (I) dudit système récupérateur 3. Selon un mode de réalisation préféré, le système récupérateur 3 comprend au moins 12 emplacements de récupération distincts (4₁ - 4₁₂) destinés la détection et/ou la quantification respective, simultanée et spécifique d'au moins 12 analytes de classes distinctes présents dans un échantillon E et au moins trois emplacements de récupération 5 destinés à un contrôle du seuil de détection ou servant de calibrateur. En outre, chacun des emplacements de récupération (4₁- 4₁₂ et 5₁-5₃) est disposé en duplicat (4_{1A} ;4_{1B} - 4_{12A} ;4_{12B}).

Il est bien entendu que la présente invention n'est en aucune façon limitée aux formes de réalisations décrites ci-dessus et que bien des modifications peuvent y être apportées sans sortir du cadre des revendications annexées.

### Modes de réalisation selon l'invention - Exemples

### Exemple 1 : Exemple de composition d'un tampon pour le mélange réactionnel et exemple d'un procédé de préparation du mélange réactionnel

**Tableau 1 :**

| Sels et additifs | Concentration finale (nM) |
|---|---|
| TRIS | 20-25 |
| HEPES | 3-10 |
| NaCl | 4-8 |
| MgCl2 | 0-2 |
| Sucre | 50-100 |
| BSA | 0-1 |
| Glycerol | 10-30 |
| Tween | 0-1 |

A ce tampon sont ajoutées les molécules de reconnaissance et/ou les ligands compétiteurs. Après l'incubation du mélange durant une nuit à 4°C, celui-ci est lyophilisé. Lors de la réalisation de l'essai, 250 µl d'échantillon à tester seront ajoutés au mélange réactionnel ainsi obtenu.

### Exemple 2 : Exemple de couplage des molécules de reconnaissance au fluorophore rhodamine B

Les récepteurs « Beta » et « Tetra » et les oligonucléotides ADN sont obtenus suivant le procédé décrit dans EP1712914A1.

Les anticorps monoclonaux sont purifiés sur colonne de protéine-A ou protéine-G en fonction de l'espèce et de l'isotype. Les anticorps sont ensuite stockés à - 20°C dans du tampon phosphate 10mM NaCl 140mM pH7,4.

La rhodamine B utilisée possède un résidu N-hydroxysuccinimidyl(NHS)-esters qui a la particularité de réagir avec les groupements amines des protéines à pH basique.

Les molécules de reconnaissance (anticorps, et/ou récepteurs) sont dialysés une nuit dans un tampon carbonate 50mM pH 8,5.
Le fluorophore est dissout dans du DMF à du 5mg/ml.

La molécule de reconnaissance et le fluorophore (la molécule de visualisation) sont mis en présence dans un rapport molaire d'environ 1/4 pendant une heure à l'abri de la lumière.

Enfin, la réaction chimique est stoppée lors de la dialyse de complexe avec un tampon phosphate 10mM pH 7,4.
D'autres types de liaison chimiques peuvent être réalisées, avec des fluorochromes possédant un groupement maléimide ou carboxyle.

D'autres types de fluorophores peuvent être utilisés, tels que le FITC, Alexa, DyLight, ...

Le couplage des molécules de reconnaissance peut aussi être réalisé avec des nanoparticules colorimétriques (nanoparticules d'or, latex, carbone...), aussi bien par couplage covalent que par adsorption électrostatique.

### Exemple 3 : Exemple de composition du mélange réactionnel et exemple d'éléments récupérateurs fixés sur le système récupérateur

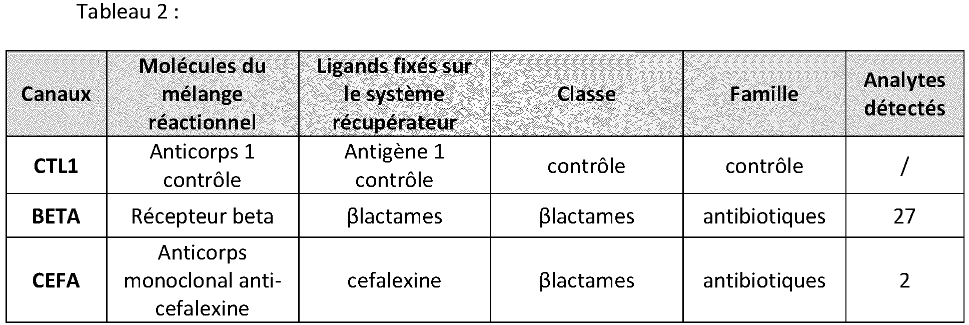

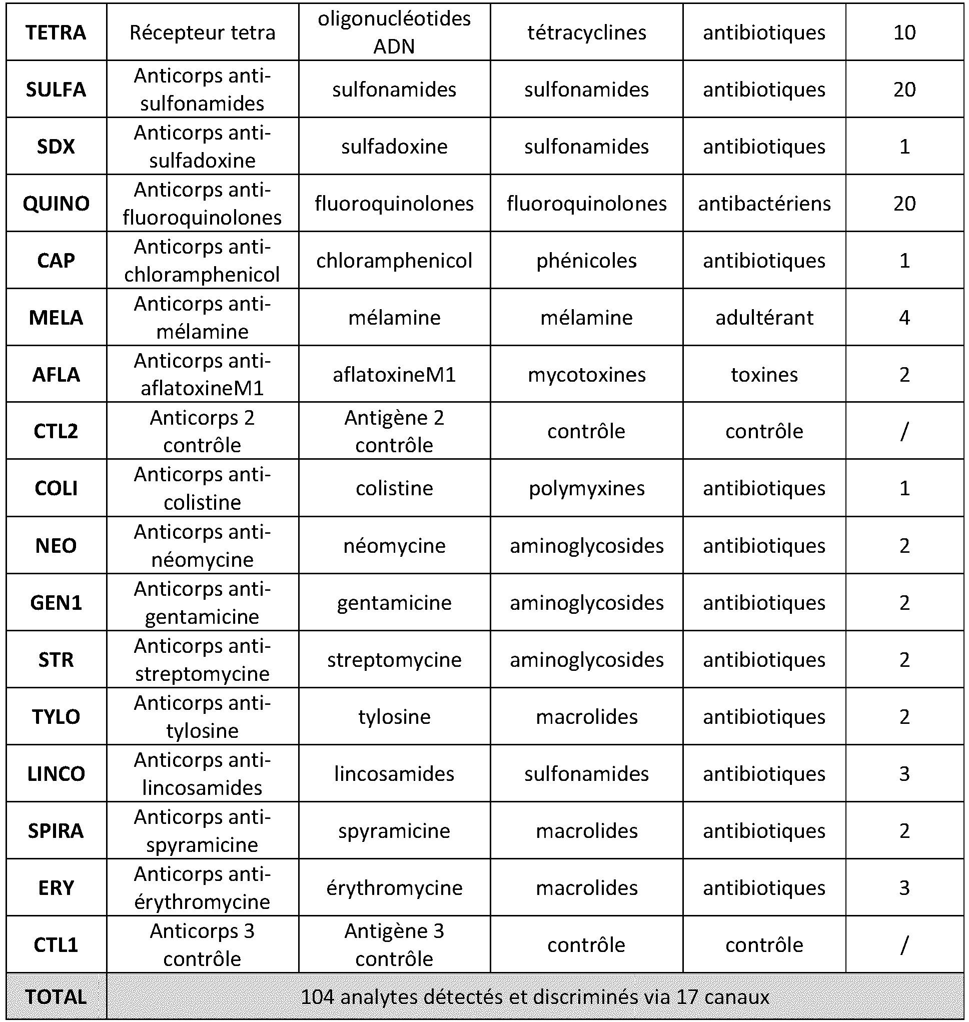

### Exemple 4 : Exemple de réalisation de l'essai et résultats obtenus

Un échantillon de lait est mis en contact avec le mélange réactionnel (comprenant le tampon et les molécules de reconnaissance et/ou les ligands compétiteurs sous forme lyophilisée) durant 3 minutes à 30°C. Ensuite, l'extrémité en amont du sens de migration du système récupérateur est plongée dans la solution (comprenant l'échantillon et le mélange réactionnel). Après une incubation de 10 minutes à 30°C, la lecture des résultats est réalisée à l'aide d'un dispositif optique.

Les résultats sont repris dans le tableau 3.

**Tableau 3 :**

| **Canaux** | **Concentrations ciblée par le test (ppb ; µg/kg)** | **Concentration (ppb ; µg/kg)** | **Signal (unité arbitraire)** | **Interprétation instrumentale** |
|---|---|---|---|---|
| **BETA** | ≥4 | 2 | 1,04 | négatif |
| | | 4 | 0,68 | positif |
| **CEFA** | ≥2 | 1 | 1,09 | négatif |
| | | 2 | 0,64 | positif |
| **TETRA** | ≥50 | 30 | 1,10 | négatif |
| | | 50 | 0,71 | positif |
| **SULFA** | ≥100 | 50 | 1,08 | négatif |
| | | 100 | 0,71 | positif |
| **SDX** | ≥100 | 50 | 1,08 | négatif |
| | | 100 | 0,69 | positif |
| **QUINO** | ≥20 | 10 | 1,29 | négatif |
| | | 20 | 0,69 | positif |
| **CAP** | ≥0,3 | 0,2 | 1,01 | négatif |
| | | 0,3 | 0,84 | positif |
| **MELA** | ≥15 | 10 | 1,11 | négatif |
| | | 15 | 0,86 | positif |
| **AFLA** | ≥0,3 | 0,1 | 1,05 | négatif |
| | | 0,3 | 0,93 | positif |
| **COLI** | ≥25 | 20 | 1,14 | négatif |
| | | 25 | 0,72 | positif |
| **NEO** | ≥1200 | 900 | 1,04 | négatif |
| | | 1200 | 0,69 | positif |
| **GEN** | ≥80 | 60 | 1,03 | négatif |
| | | 80 | 0,68 | positif |
| **STR** | ≥200 | 150 | 1,05 | négatif |
| | | 200 | 0,68 | positif |
| **TYLO** | ≥40 | 30 | 1,14 | négatif |
| | | 40 | 0,80 | positif |
| **LINCO** | ≥80 | 60 | 1,08 | négatif |
| | | 80 | 0,66 | positif |
| **SPIRA** | ≥50 | 30 | 1,05 | négatif |
| | | 50 | 0,79 | positif |
| **ERY** | ≥20 | 10 | 1,23 | négatif |
| | | 20 | 0,73 | positif |

## Revendications

1. Moyen de diagnostic immuno-chromatographique (1) pour la détection et/ou la quantification respective, simultanée et spécifique d'une pluralité d'analytes présents dans un échantillon (E) essentiellement liquide comprenant :
- au moins un mélange réactionnel (2) contenant des molécules biologiques de reconnaissance et/ou des ligands compétiteurs marqués avec au moins une molécule de visualisation ; et
- au moins un système récupérateur (3) sous la forme d'un support solide auquel se trouvent fixés des ligands compétiteurs et/ou des molécules biologiques de reconnaissance en des emplacements de récupération (4 et 5) distincts et connus qui sont disposés selon un arrangement matriciel en deux dimensions, de manière à identifier par la localisation desdits emplacements de récupération (4 et 5) sur ledit support, lesdits analytes présents dans ledit échantillon (E),
ledit moyen de diagnostic (1) étant **caractérisé en ce que**,
a) ledit arrangement matriciel en deux dimensions est défini selon un système de coordonnées présentant une première coordonnée (X) et une deuxième coordonnée (Y), tel que chaque emplacement de récupération fixé sur ledit support solide permet l'identification d'un analyte distinct et
avec, pour une même coordonnée X, plusieurs emplacements de récupération chacun comprenant des molécules biologiques de reconnaissance ou des ligands compétiteurs différents, disposés selon des coordonnées Y différentes et, avec, pour une même coordonnée Y, plusieurs emplacements de récupération chacun comprenant des molécules biologiques de reconnaissance ou des ligands compétiteurs différents, disposés selon des coordonnées X différentes;
b) pour la détection et/ou la quantification d'un analyte donné, un couple de diagnostic constitué d'un ligand compétiteur et d'une molécule biologique de reconnaissance est présent, tel que ladite molécule biologique de reconnaissance se trouve dans ledit mélange réactionnel (2) et ledit ligand compétiteur est fixé en au moins un emplacement de récupération (4), ou inversement ;
c) ladite au moins une molécule de visualisation est une molécule détectable en fluorescence ;
d) ledit mélange réactionnel (2) est présent dans un contenant, ledit contenant étant distinct et séparé dudit système récupérateur (3), l'interaction du mélange réactionnel (2) avec l'échantillon à analyser (E) étant focalisée dans ledit contenant distinct dudit système récupérateur (3) de telle sorte que ledit échantillon (E) interagit complètement avec le mélange réactionnel (2) avant que le liquide ainsi obtenu, formé du mélange réactionnel et de l'échantillon, ne soit en contact avec le support solide et donc avec les emplacements de récupération, et
e) ledit système récupérateur (3) comprend au moins 5 emplacements de récupération (4) distincts destinés à la détection et/ou la quantification respective, simultanée et spécifique d'au moins 5 analytes distincts présents dans un échantillon, et au moins un emplacement de récupération destiné à un contrôle et/ou un calibrateur.

2. Moyen de diagnostic (1) selon la revendication 1, **caractérisé en ce que** lesdits emplacements de récupération (4 et 5) sont disposés selon un arrangement matriciel en deux dimensions sous la forme de points présentant chacun un diamètre compris entre 20 µm à 2 mm, de préférence compris entre 100 à 500 µm, préférentiellement entre 250 et 400 µm.

3. Moyen de diagnostic (1) selon la revendication 1 ou 2, **caractérisé en ce que** ledit système récupérateur (3) comprend au moins 10, préférentiellement au moins 15 emplacements de récupération (4) distincts destinés à la détection et/ou la quantification respective, simultanée et spécifique d'au moins 10, préférentiellement d'au moins 15 analytes distincts présents dans un échantillon, et au moins un emplacement de récupération destiné à un contrôle et/ou un calibrateur.

4. Moyen de diagnostic (1) selon l'une quelconque des revendications 1 à 3, **caractérisé en ce que** ledit système récupérateur (3) est sous la forme d'un support solide comprend une membrane ou un ensemble de membranes.

5. Moyen de diagnostic (1) selon l'une quelconque des revendications 1 à 4, caractérisé en que ladite au moins une molécule de visualisation est fusionnée auxdites molécules biologiques de reconnaissance et/ou auxdits ligands compétiteurs via un couplage chimique et/ou génétique.

6. Moyen de diagnostic (1) selon la revendication 5, caractérisé en que ledit couplage chimique et/ou génétique est réalisé via au moins une force électrostatique, au moins un lien peptidique, au moins un gène rapporteur, ou une combinaison de ceux-ci.

7. Moyen de diagnostic (1) selon l'une quelconque des revendications 1 à 6, **caractérisé en ce que** lesdits analytes sont sélectionnés dans le groupe constitué des résidus médicamenteux, des toxines, des virus, des bactéries, des hormones, des métaux lourds, des adultérants, des allergènes et de leurs mélanges.

8. Moyen de diagnostic (1) selon la revendication 7, **caractérisé en ce que** lesdits analytes sont des résidus médicamenteux et sont choisis dans le groupe constitué des pénicillines, des céphalosporines, des tétracyclines, des sulfamides, des aminoglycosides, des aminocyclitols, des macrolides, des quinolones, des ionophores, des carbadox, des nitrofurannes, des phénicols, et de leurs mélanges.

9. Procédé pour la détection et/ou la quantification respective, simultanée et spécifique d'une pluralité d'analytes présents dans un échantillon (E) essentiellement liquide comprenant les étapes suivantes :
- mettre en contact un mélange réactionnel d'un moyen d'un diagnostic selon l'une quelconque des revendications 1 à 8 avec l'échantillon (E) pour obtenir un liquide ;
- incuber à une température comprise entre 0 et 70°C, pendant une durée inférieure ou égale à 15 minutes ;
- tremper une extrémité d'un système récupérateur d'un moyen de diagnostic selon l'une quelconque des revendications 1 à 8 dans le liquide ;
- incuber à une température comprise entre 0 et 70°C, pendant une durée inférieure ou égale à 15 minutes ; et
- interpréter qualitativement et/ou quantitativement le résultat sur le système récupérateur au moyen d'un dispositif optique.

10. Ensemble de diagnostic pour la détection et/ou la quantification respective, simultanée et spécifique d'analytes présents dans un échantillon (E) comprenant un moyen de diagnostic (1) selon l'une quelconque des revendications 1 à 8, **caractérisé en ce qu'**il comprend en outre un dispositif de lecture optique d'un support solide (3) amovible comprenant :
- un emplacement pour recevoir ledit support solide (3) ;
- une unité optique pour analyser ledit support solide (3) et comprenant :
∘ une première source lumineuse pour émettre selon une intensité d'émission et dans une première plage de longueur d'onde un premier faisceau lumineux vers ledit emplacement ;
∘ un système d'imagerie comprenant un détecteur optique pour fournir une image d'une zone de visualisation, ladite zone de visualisation comprenant au moins une portion dudit emplacement ;
∘ un filtre pour filtrer une plage de longueur d'onde définie, et positionné entre l'emplacement et ledit système d'imagerie ;
- des moyens de communications pour obtenir une information relative à un support solide (3) ;
- des moyens de sélection pour :
∘ sélectionner à partir d'une liste d'analytes prédéfinis correspondant auxdits emplacements de récupération fixés sur le support solide (3), une sélection d'analytes à détecter et/ou à quantifier pour ledit échantillon à partir d'un même support solide (3) ;
- des moyens de traitement d'image de ladite image pour:
∘ déterminer, à partir de l'information relative audit support solide (3) à lire, un nombre fini de sous-ensembles de ladite image, chaque sous-ensemble correspondant à un analyte ;
∘ fournir des données relatives à des intensités lumineuses issues desdits sous-ensembles;
- des moyens de détermination pour :
∘ calculer, pour chaque sous-ensemble correspondant à un analyte sélectionné dans ladite sélection d'analytes une intensité de sous-ensemble ;
∘ déterminer sur la base de ladite intensité de sous-ensemble des informations d'analytes dudit échantillon pour chaque sous-ensemble correspondant à un analyte sélectionné dans ladite sélection d'analytes ;
- des moyens de transmission configurés pour transmettre ladite information d'analytes dudit échantillon analysé pour chaque sous-ensemble correspondant à un analyte sélectionné dans ladite sélection d'analytes.

11. Ensemble de diagnostic selon la revendication 10, **caractérisé en ce que** le dispositif optique comprend en outre :
- des moyens permettant de lire un profil de sélection ;
et **en ce que**, lesdits moyens de sélection sont configurés pour réaliser ladite sélection d'analytes sur la base dudit profil de sélection.

12. Ensemble de diagnostic pour la détection et/ou la quantification respective, simultanée et spécifique d'analytes présents dans un échantillon (E) comprenant un moyen de diagnostic (1) selon l'une quelconque des revendications 1 à 8, **caractérisé en ce qu'**il comprend en outre un dispositif de lecture optique d'un support solide amovible comprenant :
- un emplacement pour recevoir ledit support solide (3) ;
- une unité optique pour analyser ledit support solide (3) et comprenant :
∘ une première source lumineuse pour émettre selon une intensité d'émission et dans une première plage de longueur d'onde un premier faisceau lumineux vers ledit emplacement ;
∘ un capteur d'intensité lumineuse pour mesurer l'intensité d'émission émise par ladite première source lumineuse ;
∘ un moyen de rétrocontrôle pour moduler ladite intensité d'émission de ladite première source lumineuse en fonction de l'intensité d'émission mesurée par ledit capteur d'intensité lumineuse de sorte que ladite première source lumineuse émette une intensité cible ;
∘ un système d'imagerie comprenant un détecteur optique pour fournir une image d'une zone de visualisation, ladite zone de visualisation comprenant au moins une portion dudit emplacement ;
∘ un filtre pour filtrer une plage de longueur d'onde définie, et positionné entre l'emplacement et ledit système d'imagerie ;
- des moyens de traitement d'image de ladite image pour :
∘ déterminer un nombre fini de sous-ensembles de ladite image,
∘ fournir des données relatives à des intensités lumineuses issues desdits sous-ensembles ;
- des moyens de détermination pour:
∘ calculer, pour chaque sous-ensemble, une intensité de sous-ensemble, et
- des moyens de transmission pour transmettre une information relative à ladite intensité de sous-ensemble pour chaque sous-ensemble.

13. Utilisation d'un moyen de diagnostic (1) selon l'une quelconque des revendications 1 à 8 pour la détection et/ou la quantification respective, simultanée et spécifique d'au moins 5 analytes présents dans un échantillon (E), de préférence d'au moins 10, préférentiellement d'au moins 15 analytes appartenant à des classes distinctes, faisant partie de familles d'analytes différentes ou non.

14. Utilisation d'un ensemble de diagnostic selon l'une quelconque des revendications 10 à 12, pour la détection et/ou la quantification respective, simultanée et spécifique de classes d'au moins 5 analytes présents dans un échantillon (E), de préférence d'au moins 10, préférentiellement d'au moins 15 analytes appartenant à des classes distinctes, faisant partie de familles d'analytes différentes ou non.

## Patentansprüche

1. Immunchromatographisches Diagnosemittel (1) zum jeweiligen, gleichzeitigen und spezifischen Nachweis und/oder der Quantifizierung einer Vielzahl von Analyten, die in einer im Wesentlichen flüssigen Probe (E) vorhanden sind, umfassend:
- mindestens ein Reaktionsgemisch (2), das biologische Erkennungsmoleküle und/oder konkurrierende Liganden enthält, die mit mindestens einem Molekül zur Sichtbarmachung markiert sind; und
- mindestens ein Auffangsystem (3) in Form eines festen Trägers, an dem konkurrierende Liganden und/oder biologische Erkennungsmoleküle an verschiedenen und bekannten Auffangstellen (4 und 5), die in einer zweidimensionalen Matrixanordnung angeordnet sind, fixiert sind, um durch die Lokalisierung der Auffangstellen (4 und 5) auf dem Träger die Analyten, die in der Probe (E) vorhanden sind, zu identifizieren,
wobei das Diagnosemittel (1) **dadurch gekennzeichnet ist, dass**
a) die zweidimensionale Matrixanordnung gemäß einem Koordinatensystem definiert ist, das eine erste Koordinate (X) und eine zweite Koordinate (Y) aufweist, derart, dass jede Auffangstelle, die an dem festen Träger fixiert ist, die Identifizierung eines verschiedenen Analyten ermöglicht, und
wobei bei derselben X-Koordinate mehrere Auffangstellen, die jeweils unterschiedliche biologische Erkennungsmoleküle oder konkurrierende Liganden umfassen, entlang unterschiedlicher Y-Koordinaten angeordnet sind, und
wobei bei derselben *Y*-Koordinate mehrere Auffangstellen, die jeweils unterschiedliche biologische Erkennungsmoleküle oder konkurrierende Liganden umfassen, entlang unterschiedlicher X-Koordinaten angeordnet sind;
b) für den Nachweis und/oder die Quantifizierung eines gegebenen Analyten ein Diagnosepaar, das aus einem konkurrierenden Liganden und einem biologischen Erkennungsmolekül besteht, derart vorliegt, dass sich das biologische Erkennungsmolekül in dem Reaktionsgemisch (2) befindet und der konkurrierende Ligand an mindestens einer Auffangstelle (4) fixiert ist, oder umgekehrt;
c) das mindestens eine Molekül zur Sichtbarmachung ein durch Fluoreszenz nachweisbares Molekül ist;
d) das Reaktionsgemisch (2) in einem Behälter vorliegt, wobei der Behälter vom Auffangsystem (3) verschieden und getrennt ist, wobei die Wechselwirkung des Reaktionsgemisches (2) mit der zu analysierenden Probe (E) in dem vom Auffangsystem (3) verschiedenen Behälter derart konzentriert ist, dass die Probe (E) vollständig mit dem Reaktionsgemisch (2) wechselwirkt, bevor die so erhaltene Flüssigkeit, die aus dem Reaktionsgemisch und der Probe gebildet ist, mit dem festen Träger und somit mit den Auffangstellen in Kontakt gelangt, und
e) das Auffangsystem (3) mindestens 5 verschiedene Auffangstellen (4), die zum jeweiligen, gleichzeitigen und spezifischen Nachweis und/oder der Quantifizierung von mindestens 5 verschiedenen Analyten, die in einer Probe vorhanden sind, bestimmt sind, und mindestens eine Auffangstelle, die für eine Kontrolle und/oder einen Kalibrator bestimmt ist, umfasst.

2. Diagnosemittel (1) nach Anspruch 1, **dadurch gekennzeichnet, dass** die Auffangstellen (4 und 5) in einer zweidimensionalen Matrixanordnung in Form von Punkten angeordnet sind, die jeweils einen Durchmesser im Bereich zwischen 20 µm bis 2 mm, bevorzugt im Bereich zwischen 100 und 500 µm, vorzugsweise zwischen 250 und 400 µm, aufweisen.

3. Diagnosemittel (1) nach Anspruch 1 oder 2, **dadurch gekennzeichnet, dass** das Auffangsystem (3) mindestens 10, vorzugsweise mindestens 15 verschiedene Auffangstellen (4), die zum jeweiligen, gleichzeitigen und spezifischen Nachweis und/oder der Quantifizierung von mindestens 10, vorzugsweise mindestens 15 verschiedenen Analyten, die in einer Probe vorhanden sind, bestimmt sind, und mindestens eine Auffangstelle, die für eine Kontrolle und/oder einen Kalibrator bestimmt ist, umfasst.

4. Diagnosemittel (1) nach einem der Ansprüche 1 bis 3, **dadurch gekennzeichnet, dass** das Auffangsystem (3) in Form eines festen Trägers vorliegt, der eine Membran oder eine Membrananordnung umfasst.

5. Diagnosemittel (1) nach einem der Ansprüche 1 bis 4, **dadurch gekennzeichnet, dass** das mindestens eine Molekül zur Sichtbarmachung über eine chemische und/oder genetische Kopplung mit den biologischen Erkennungsmolekülen und/oder den konkurrierenden Liganden fusioniert ist.

6. Diagnosemittel (1) nach Anspruch 5, **dadurch gekennzeichnet, dass** die chemische und/oder genetische Kopplung über mindestens eine elektrostatische Kraft, mindestens eine Peptidbindung, mindestens ein Reportergen oder eine Kombination davon hergestellt ist.

7. Diagnosemittel (1) nach einem der Ansprüche 1 bis 6, **dadurch gekennzeichnet, dass** die Analyten ausgewählt sind aus der Gruppe bestehend aus Arzneimittelrückständen, Toxinen, Viren, Bakterien, Hormonen, Schwermetallen, Streckmitteln, Allergenen und Gemischen davon.

8. Diagnosemittel (1) nach Anspruch 7, **dadurch gekennzeichnet, dass** die Analyten Arzneimittelrückstände sind und ausgewählt sind aus der Gruppe bestehend aus Penicillinen, Cephalosporinen, Tetracyclinen, Sulfonamiden, Aminoglykosiden, Aminocyclitolen, Makroliden, Chinolonen, Ionophoren, Carbadox, Nitrofuranen, Phenicolen und Gemischen davon.

9. Verfahren zum jeweiligen, gleichzeitigen und spezifischen Nachweis und/oder der Quantifizierung einer Vielzahl von Analyten, die in einer im Wesentlichen flüssigen Probe (E) vorhanden sind, das die folgenden Schritte umfasst:
- Inkontaktbringen eines Reaktionsgemisches eines Diagnosemittels nach einem der Ansprüche 1 bis 8 mit der Probe (E), um eine Flüssigkeit zu erhalten;
- Inkubieren bei einer Temperatur im Bereich zwischen 0 und 70 °C über eine Dauer von kleiner oder gleich 15 Minuten;
- Eintauchen eines Endes eines Auffangsystems eines Diagnosemittels nach einem der Ansprüche 1 bis 8 in die Flüssigkeit;
- Inkubieren bei einer Temperatur im Bereich zwischen 0 und 70 °C über eine Dauer von kleiner oder gleich 15 Minuten; und
- qualitatives und/oder quantitatives Auswerten des Ergebnisses auf dem Auffangsystem mittels einer optischen Vorrichtung.

10. Diagnoseanordnung für den jeweiligen, gleichzeitigen und spezifischen Nachweis und/oder die Quantifizierung von Analyten, die in einer Probe (E) vorhanden sind, die ein Diagnosemittel (1) nach einem der Ansprüche 1 bis 8 umfasst, **dadurch gekennzeichnet, dass** sie weiter eine optische Lesevorrichtung für einen abnehmbaren festen Träger (3) umfasst, welche umfasst:
- eine Stelle, um den festen Träger (3) aufzunehmen;
- eine optische Einheit, um den festen Träger (3) zu analysieren, umfassend:
∘ eine erste Lichtquelle, um in einer Emissionsstärke und in einem ersten Wellenlängenbereich einen ersten Lichtstrahl in Richtung der Stelle zu emittieren;
∘ ein Bildgebungssystem, das einen optischen Detektor umfasst, um ein Bild eines Sichtbarmachungsbereichs zu liefern, wobei der Sichtbarmachungsbereich mindestens einen Abschnitt der Stelle umfasst;
∘ einen Filter, um einen definierten Wellenlängenbereich zu filtern, der zwischen der Stelle und dem Bildgebungssystem positioniert ist;
- Kommunikationsmittel, um eine Information in Bezug auf einen festen Träger (3) zu erhalten;
- Auswahlmittel, um:
∘ anhand einer Liste vordefinierter Analyten, die den auf dem festen Träger (3) fixierten Auffangstellen entsprechen, eine Auswahl von Analyten auszuwählen, die anhand desselben festen Trägers (3) bei der Probe nachgewiesen und/oder quantifiziert werden sollen;
- Mittel zur Bildverarbeitung des Bildes, um:
∘ anhand der Information in Bezug auf den festen Träger (3), der ausgelesen werden soll, eine endliche Anzahl von Teilmengen des Bildes zu bestimmen, wobei jede Teilmenge einem Analyten entspricht;
∘ Daten in Bezug auf Lichtstärken, die von den Teilmengen stammen, zu liefern;
- Bestimmungsmittel, um:
∘ für jede Teilmenge, die einem in der Analytenauswahl ausgewählten Analyten entspricht, eine Teilmengenstärke zu berechnen;
∘ auf Basis der Teilmengenstärke Informationen zu Analyten der Probe für jede Teilmenge, die einem in der Analytenauswahl ausgewählten Analyten entspricht, zu bestimmen;
- Übertragungsmittel, die dafür konfiguriert sind, die Information zu Analyten der analysierten Probe für jede Teilmenge, die einem in der Analytenauswahl ausgewählten Analyten entspricht, zu übertragen.

11. Diagnoseanordnung nach Anspruch 10, **dadurch gekennzeichnet, dass** die optische Vorrichtung weiter umfasst:
- Mittel, die es ermöglichen, ein Auswahlprofil zu lesen;
und dadurch, dass die Auswahlmittel dafür konfiguriert sind, die Auswahl von Analyten auf Basis des Auswahlprofils auszuführen.

12. Diagnoseanordnung für den jeweiligen, gleichzeitigen und spezifischen Nachweis und/oder die Quantifizierung von Analyten, die in einer Probe (E) vorhanden sind, die ein Diagnosemittel (1) nach einem der Ansprüche 1 bis 8 umfasst, **dadurch gekennzeichnet, dass** sie weiter eine optische Lesevorrichtung für einen abnehmbaren festen Träger umfasst, welche umfasst:
- eine Stelle, um den festen Träger (3) aufzunehmen;
- eine optische Einheit, um den festen Träger (3) zu analysieren, umfassend:
∘ eine erste Lichtquelle, um in einer Emissionsstärke und in einem ersten Wellenlängenbereich einen ersten Lichtstrahl in Richtung der Stelle zu emittieren;
∘ einen Lichtstärkesensor, um die von der ersten Lichtquelle emittierte Emissionsstärke zu messen;
∘ ein Rückkopplungsmittel, um die Emissionsstärke der ersten Lichtquelle in Abhängigkeit von der durch den Lichtstärkesensor gemessenen Emissionsstärke so zu modulieren, dass die erste Lichtquelle eine Zielstärke emittiert;
∘ ein Bildgebungssystem, das einen optischen Detektor umfasst, um ein Bild eines Sichtbarmachungsbereichs zu liefern, wobei der Sichtbarmachungsbereich mindestens einen Abschnitt der Stelle umfasst;
∘ einen Filter, um einen definierten Wellenlängenbereich zu filtern, der zwischen der Stelle und dem Bildgebungssystem positioniert ist;
- Mittel zur Bildverarbeitung des Bildes, um:
∘ eine endliche Anzahl von Teilmengen des Bildes zu bestimmen,
∘ Daten in Bezug auf Lichtstärken, die von den Teilmengen stammen, zu liefern;
- Bestimmungsmittel, um:
∘ für jede Teilmenge eine Teilmengenstärke zu berechnen, und
- Übertragungsmittel, um eine Information in Bezug auf die Teilmengenstärke für jede Teilmenge zu übertragen.

13. Verwendung eines Diagnosemittels (1) nach einem der Ansprüche 1 bis 8 für den jeweiligen, gleichzeitigen und spezifischen Nachweis und/oder die Quantifizierung von mindestens 5 Analyten, die in einer Probe (E) vorhanden sind, bevorzugt mindestens 10, vorzugsweise mindestens 15 Analyten, die zu verschiedenen Klassen gehören, die Teil unterschiedlicher Analytenfamilien sind oder nicht.

14. Verwendung einer Diagnoseanordnung nach einem der Ansprüche 10 bis 12 für den jeweiligen, gleichzeitigen und spezifischen Nachweis und/oder die Quantifizierung der Klassen von mindestens 5 Analyten, die in einer Probe (E) vorhanden sind, bevorzugt mindestens 10, vorzugsweise mindestens 15 Analyten, die zu verschiedenen Klassen gehören, die Teil unterschiedlicher Analytenfamilien sind oder nicht.

## Claims

1. Immuno-chromatographic diagnosis means (1) for respectively, simultaneously and specifically detecting and/or quantifying a plurality of analytes present in an essentially liquid sample (E) comprising:
- at least one reaction mixture (2) containing recognition biological molecules and/or competitive ligands labelled with at least one visualisation molecule; and
- at least one recovery system (3) in the form of a solid support to which are bonded competitive ligands and/or recognition biological molecules at distinct and known recovery locations (4 and 5) which are arranged according to a two-dimensional matrix arrangement, so as to identify, by the localisation of said recovery locations (4 and 5) on said support, said analytes present in said sample (E),
said diagnosis means (1) being **characterised in that**,
a) said two-dimensional matrix arrangement is defined according to a system of coordinates having a first coordinate (X) and a second coordinate (Y), such that each recovery location bonded on said solid support makes it possible to identify a distinct analyte and
with, for one same coordinate *X,* several recovery locations each comprising different recognition biological molecules or competitive ligands, arranged along different coordinates *Y* and,
with, for one same coordinate *Y,* several recovery locations each comprising different recognition biological molecules or competitive ligands, arranged along different coordinates X;
b) for the detection and/or the quantification of a given analyte, a diagnosis couple consisting of a competitive ligand and a recognition biological molecule is present, such that said recognition biological molecule is found in said reaction mixture (2) and said competitive ligand is bonded at at least one recovery location (4) or conversely;
c) said at least one visualisation molecule is a molecule which is detectable in fluorescence; and
d) said reaction mixture (2) is present in a container, said container being separate from said recovery system (3); the interaction of the reaction mixture (2) with the sample to be analysed (E) being focalised in said container separate from said recovery system (3) in such a way that the sample (E) interacts completely with the reaction mixture (2) before the liquid thus obtained, formed from the reaction mixture and from the sample, is in contact with the solid support and therefore with the recovery locations, and
e) said recovery system (3) comprises at least 5 distinct recovery locations (4) intended for respectively, simultaneously and specifically detect and/or quantify at least 5 distinct analytes present in a sample, and at least one recovery location intended for a control and/or a calibrator location.

2. Diagnosis means (1) according to claim 1, **characterised in that** said recovery locations (4 and 5) are arranged according to a two-dimensional matrix arrangement in the form of points each having a diameter of between 20µm to 2mm, preferably of between 100 to 500µm, preferably between 250 and 400µm.

3. Diagnosis means (1) according to claim 1 or 2, **characterised in that** said recovery system (3) comprises at least 10, preferably at least 15 distinct recovery locations (4), intended to respectively, simultaneously and specifically detect and/or quantify at least 10, preferably at least 15 distinct analytes present in a sample, and at least one recovery location intended for a control and/or a calibrator.

4. Diagnosis means (1) according to any one of claims 1 to 3, **characterised in that** said recovery system (3) in the form of a solid support comprises a membrane or a set of membranes.

5. Diagnosis means (1) according to any one of claims 1 to 4, **characterised in that** said at least one visualisation molecule is fused to said recognition biological molecules and/or to said competitive ligands via a chemical and/or genetic coupling.

6. Diagnosis means (1) according to claim 5, **characterised in that** said chemical and/or genetic coupling is carried out via at least one electrostatic force, at least one peptide bond, at least one reporter gene, or a combination thereof.

7. Diagnosis means (1) according to any one of claims 1 to 6, **characterised in that** said analytes are selected from the group consisting of drug residues, toxins, viruses, bacteria, hormones, heavy metals, adulterants, allergens and the mixtures thereof.

8. Diagnosis means (1) according to claim 7, **characterised in that** said analytes are drug residues and are selected from the group consisting of penicillins, cephalosporines, tetracyclines, sulphonamides, aminoglycosides, aminocyclitols, macrolides, quinolones, ionophores, carbadox, nitrofurans, phenicols, and the mixtures thereof.

9. Method for respectively, simultaneously and specifically detecting and/or quantifying a plurality of analytes present in an essentially liquid sample (E) comprising the following steps:
- contacting a reaction mixture of a diagnosis means according to any one of claims 1 to 8 with the sample (E) to obtain a liquid;
- incubating at a temperature of between 0 and 70°C, for a duration less than or equal to 15 minutes;
- soaking an end of a recovery system of a diagnosis means according to any one of claims 1 to 8 in the liquid;
- incubating at a temperature of between 0 and 70°C, for a duration less than or equal to 15 minutes; and
- interpreting qualitatively and/or quantitatively the result on the recovery system by means of an optical device.

10. Diagnosis set for respectively, simultaneously and specifically detecting and/or quantifying analytes present in a sample (E) comprising a diagnosis means (1) according to any one of claims 1 to 8, **characterised in that** it further comprises a device for optically reading a removable solid support (3), comprising:
- a placement to receive said solid support (3);
- an optical unit to analyse said solid support (3) and comprising:
∘ a first light source to emit according to an emission intensity and in a first wavelength range, a first light beam to said placement;
∘ an imaging system comprising an optical detector to provide an image of a visualisation zone, said visualisation zone comprising at least one portion of said placement;
∘ a filter to filter a defined wavelength range defined, and positioned between the placement and said imaging system;
- communication means to obtain an item of information relative to a solid support (3);
- selection means to:
∘ select from a list of predefined analytes corresponding to said recovery locations bonded on the solid support (3), a selection of analytes to be detected and/or to be quantified for said sample from one same solid support (3);
- image processing means of said image to:
∘ determine, from the information relating to said solid support (3) to be read, a finite number of subassemblies of said image, each subassembly corresponding to an analyte;
∘ provide data relating to light intensities coming from said subassemblies;
- determination means to:
∘ calculate, for each subassembly corresponding to an analyte selected in said selection of analytes, a subassembly intensity;
∘ determine, based on said subassembly intensity, analyte information from said sample for each subassembly corresponding to an analyte selected in said selection of analytes;
- transmission means configured to transmit said analyte information from said sample analysed for each subassembly corresponding to an analyte selected in said selection of analytes.

11. Diagnosis set according to claim 10, **characterised in that** the optical device further comprises:
- means making it possible to read a selection profile;
and **in that**, said selection means are configured to carry out said selection of analytes based on said selection profile.

12. Diagnosis set for respectively, simultaneously and specifically detecting and/or quantifying analytes present in a sample (E) comprising a diagnosis means (1) according to any one of claims 1 to 8, **characterised in that** it further comprises a device for optically reading a removable solid support, comprising:
- a placement to receive said solid support (3);
- an optical unit to analyse said solid support (3) and comprising:
- a placement to receive said solid support (3);
- an optical unit to analyse said solid support (3) and comprising:
∘ a first light source to emit according to an emission intensity and in a first wavelength range, a first light beam to said placement;
∘ a light intensity sensor to measure the emission intensity emitted by said first light source;
∘ a feedback means to modulate said emission intensity of said first light source according to the emission intensity measured by said light intensity sensor such that said first light source emits a target intensity;
∘ an imaging system comprising an optical detector to provide an image of a visualisation zone, said visualisation zone comprising at least one portion of said placement;
∘ a filter to filter a defined wavelength range, and positioned between the placement and said imaging system;
- image processing means of said image to:
∘ determine a finite number of subassemblies of said image,
∘ provide data relating to light intensities coming from said subassemblies;
- determination means to:
∘ calculate, for each subassembly, a subassembly intensity, and
- transmission means to transmit an item of information relating to said subassembly intensity for each subassembly.

13. Use of a diagnosis means (1) according to any one of claims 1 to 8, for respectively, simultaneously and specifically detecting and/or quantifying at least 5 analytes present in a sample (E), preferably at least 10, preferably at least 15 analytes belonging to separate classes, forming part of families of analytes, which are different or not.

14. Use of a diagnosis set according to any one of claims 10 to 12, for respectively, simultaneously and specifically detecting and/or quantifying classes of at least 5 analytes present in a sample (E), preferably at least 10, preferably at least 15 analytes belonging to separate classes, forming part of families of analytes, which are different or not.
